# EUROPEAN PATENT SPECIFICATION

(11) **EP 3 463 434 B1**
(45) Date of publication and mention of the grant of the patent: **25.12.2024**
(21) Application number: 17728191.2
(22) Date of filing: 06.06.2017
(51) Int. Cl.: A61K 39/00, A61K 35/76, A61P 35/00, A61K 31/00, A61K 39/395

(54) **IMMUNOTHERAPY FOR CANCER**
IMMUNTHERAPIE GEGEN KREBS
IMMUNOTHÉRAPIE CONTRE LE CANCER

(30) Priority: 06.06.2016 EP 16020223
(43) Date of publication of application: 10.04.2019
(73) Proprietor: Excoso a.s., 190 00 Prague 9 (CZ)
(72) Inventor: ADKINS, Irena, 25073 Prezletice (CZ); PODZIMKOVA, Nada, 37701 Jindrichuv Hradec (CZ); PALATA, Ondrej, 15000 Prague 5 (CZ); SPÍSEK, Radek, 15500 Prague 5 (CZ)
(74) Representative: Maiwald GmbH
(86) International application number: PCT/EP2017/063697
(87) International publication number: WO 2017/211816

(56) References cited:
- HUANG A ET AL: "Increased CD14<+>HLA-DR<-/low> myeloid-derived suppressor cells correlate with extrathoracic metastasis and poor response to chemotherapy in non-small cell lung cancer patients", CANCER IMMUNOLOGY, IMMUNOTHERAPY, vol. 62, no. 9, September 2013 (2013-09-01), pages 1439 - 1451, XP055238898, ISSN: 0340-7004, DOI: 10.1007/s00262-013-1450-6
- UGEL S ET AL: "Therapeutic targeting of myeloid-derived suppressor cells", CURRENT OPINION IN PHARMACOLOGY, vol. 9, no. 4, August 2009 (2009-08-01), pages 470 - 481, XP026587082, ISSN: 1471-4892, DOI: 10.1016/J.COPH.2009.06.014
- TALMADGE J E & GABRILOVICH D I: "History of myeloid-derived suppressor cells", NATURE REVIEWS. CANCER, vol. 13, no. 10, October 2013 (2013-10-01), pages 739 - 752, XP055397114, ISSN: 1474-175X, DOI: 10.1038/nrc3581
- WESOLOWSKI R ET AL: "Myeloid derived suppressor cells - a new therapeutic target in the treatment of ca", JOURNAL FOR IMMUNOTHERAPY OF CANCER, vol. 1, no. 1, 10, 15 July 2013 (2013-07-15), XP021158211, ISSN: 2051-1426, DOI: 10.1186/2051-1426-1-10
- DRAGHICIU O ET AL: "Myeloid derived suppressor cells-An overview of combat strategies to increase immunotherapy efficacy", ONCOIMMUNOLOGY, vol. 4, no. 1, E954829, 2 January 2015 (2015-01-02), XP055397115, ISSN: 2162-4011, DOI: 10.4161/21624011.2014.954829
- KUSMARTSEV S ET AL: "All-trans-Retinoic Acid Eliminates Immature Myeloid Cells from Tumor-bearing Mice and Improves the Effect of Vaccination", CANCER RESEARCH, vol. 63, 1 August 2003 (2003-08-01), pages 4441 - 4449, XP055397147
- WEISS T ET AL: "The efficacy of an IL-1alpha vaccine depends on IL-1RI availability and concomitant myeloid-derived suppressor cell reduction", JOURNAL OF IMMUNOTHERAPY, vol. 32, no. 6, 30 June 2009 (2009-06-30), pages 552 - 564, XP009500071, ISSN: 1537-4513, DOI: 10.1097/CJI.0B013E31819B7B9E
- ICLOZAN C ET AL: "Therapeutic regulation of myeloid-derived suppressor cells and immune response to cancer vaccine in patients with extensive stage small cell lung cancer", CANCER IMMUNOLOGY, IMMUNOTHERAPY, vol. 62, no. 5, 16 April 2013 (2013-04-16), pages 909 - 918, XP055397151, ISSN: 0340-7004, DOI: 10.1007/s00262-013-1396-8
- DE SANTO C ET AL: "Nitroaspirin corrects immune dysfunction in tumor-bearing hosts and promotes tumor eradication by cancer vaccination", PROCEEDINGS NATIONAL ACADEMY OF SCIENCES, vol. 102, no. 11, 15 March 2005 (2005-03-15), pages 4185 - 4190, XP055397155, ISSN: 0027-8424, DOI: 10.1073/pnas.0409783102
- ZEYTIN H E ET AL: "Combination of a Poxvirus-Based Vaccine with a Cyclooxygenase-2 Inhibitor (Celecoxib) Elicits Antitumor Immunity and Long-Term Survival in CEA.Tg/MIN Mice", CANCER RESEARCH, vol. 64, no. 10, 15 May 2004 (2004-05-15), pages 3668 - 3678, XP055397159, ISSN: 0008-5472, DOI: 10.1158/0008-5472.CAN-03-3878
- MELANI C ET AL: "Amino-Biphosphonate Mediated MMP-9 Inhibition Breaks the Tumor-Bone Marrow Axis Responsible for Myeloid-Derived Suppressor Cell Expansion and Macrophage Infiltration in Tumor Stroma", CANCER RESEARCH, vol. 67, no. 23, 1 December 2007 (2007-12-01), pages 11438 - 11446, XP055099600, ISSN: 0008-5472, DOI: 10.1158/0008-5472.CAN-07-1882
- KO H-J ET AL: "A Combination of Chemoimmunotherapies Can Efficiently Break Self-Tolerance and Induce Antitumor Immunity in a Tolerogenic Murine Tumor Model", CANCER RESEARCH, vol. 67, no. 15, 1 August 2007 (2007-08-01), pages 7477 - 7486, XP055049594, ISSN: 0008-5472, DOI: 10.1158/0008-5472.CAN-06-4639
- TSENG C-W ET AL: "Pretreatment with Cisplatin Enhances E7-Specific CD8+ T-Cell-Mediated Antitumor Immunity Induced by DNA Vaccination", CLINICAL CANCER RESEARCH, vol. 14, no. 10, 15 May 2008 (2008-05-15), pages 3185 - 3192, XP055397164, ISSN: 1078-0432, DOI: 10.1158/1078-0432.CCR-08-0037
- NAGARAJ S ET AL: "Anti-inflammatory Triterpenoid Blocks Immune Suppressive Function of MDSCs and Improves Immune Response in Cancer", CLINICAL CANCER RESEARCH, vol. 16, no. 6, 9 March 2010 (2010-03-09), pages 1812 - 1823, XP055397166, ISSN: 1078-0432, DOI: 10.1158/1078-0432.CCR-09-3272
- BOSE A ET AL: "Sunitinib facilitates the activation and recruitment of therapeutic anti-tumor immunity in concert with specific vaccination", INTERNATIONAL JOURNAL OF CANCER, vol. 129, no. 9, November 2011 (2011-11-01), pages 2158 - 2170, XP055397168, ISSN: 0020-7136, DOI: 10.1002/ijc.25863
- ISHIZAKI H ET AL: "Modified vaccinia Ankara expressing survivin combined with gemcitabine generates specific antitumor effects in a murine pancreatic carcinoma model", CANCER IMMUNOLOGY, IMMUNOTHERAPY, vol. 60, no. 1, 20 October 2010 (2010-10-20), pages 99 - 109, XP019877350, ISSN: 1432-0851, DOI: 10.1007/S00262-010-0923-0
- NAKASHIMA H ET AL: "A Novel Combination Immunotherapy for Cancer by IL-13R[alpha]2-Targeted DNA Vaccine and Immunotoxin in Murine Tumor Models", JOURNAL OF IMMUNOLOGY, vol. 187, no. 10, 17 October 2011 (2011-10-17), pages 4935 - 4946, XP055354947, ISSN: 0022-1767, DOI: 10.4049/jimmunol.1102095

## Description

Lung cancer belongs to one of the most frequently diagnosed cancers and is the leading cause of mortality among malignant diseases with a 5-year survival of 5-15 %. In the US non-small cell lung cancer (NSCLC) accounts for over 85% cases of lung cancers and is frequently diagnosed in advanced stage patients, commonly with metastatic disease (Derman et al., 2015). NSCLC can be divided according to histology into three subtypes: adenocarcinoma (AC, 50%), squamous cell carcinoma (SCC, 30%) and large cell carcinoma (5%). Histological classification of the different type of NSCLC tumors has been updated by the World Health Organization in 2015 (Travis et al., 2015). Further, differently methylated genes have been described to distinguish between AC and SCC (Huang et al., 2016). Small cell carcinoma encompasses the remaining 15% of lung cancer cases. The incidence and mortality of lung cancer remain high. Therefore there is an urgent need for the development of improved therapies possibly using immunotherapeutic strategies.

Despite enormous efforts in the field of immunotherapies so far only very few treatment modalities have actually reached the market. This may be due to a still incomplete understanding of the relevance of immune cell infiltration in tumors and the mechanisms behind the immune suppression commonly observed in cancer patients.

Immune suppression is a main feature of myeloid-derived suppressor cells (MDSC or MDSCs). The main targets of MDSCs are T cells. The main factors involved in MDSC-mediated immune suppression include for example arginase (ARG1), iNOS, TGFb, IL10, COX2, indoleamine 2,3-dioxygenase (IDO), sequestration of cysteine, decrease of L-selectin expression by T cells. Monocytic MDSCs (M-MDSCs) suppress T-cell responses both in antigen-specific and non-specific manners, utilizing mechanisms associated with production of NO and cytokines (Gabrilovich et al., 2012). Polymorphonuclear MDSCs (PMN-MDSCs) are capable of suppressing immune responses primarily in an antigen-specific manner, inducing antigen-specific T-cell tolerance (Koehn et al., 2015, Marigo et al., 2010). Reactive oxygen species (ROS) production is essential for this ability. Reaction of NO with superoxide generates peroxynitrite (PNT), which directly inhibits T cells by nitrating T-cell receptors and reducing their responsiveness to cognate antigen-MHC complexes (Nagaraj et al., 2007). PNT also reduces the binding of antigenic peptides to MHC molecules on tumor cells (Lu et al., 2011b) and blocks T-cell migration by nitrating T-cell-specific chemokines (Molon et al., 2011).

A second class of immune suppressive cells in the tumor microenvironment are regulatory T cells (Tregs), which are reported to play a crucial role in the inhibition of immune responses in lung cancer (Domagala-Kulawik et al., 2014).

Huang et al. Cancer Immunol Immmunother 62(9):1439-1451 (2013) disclose that increased CD14⁺HLA-DR^{-/low} myeloid-derived suppressor cells correlate with extrathoracic metastasis and poor response to chemotherapy in non-small cell lung cancer patients.

Still, the tumor microenvironment in various tumors or tumor subtypes is poorly understood. Surprisingly it was found in the course of the present invention that actually MDSCs play the dominant role of immune suppression in a subtype of lung cancer, namely SCC. Therefore, the invention relates to immunogenic compositions for the treatment of cancer, more specifically a treatment limiting the immunosuppressive microenvironment of tumors for patients suffering from SCC or stratified patients suffering from NSCLC.

### Definitions

"Immunogenic composition" refers to a composition which induces an immune response in an individual. Such immunogenic compositions are typically all kinds of vaccines, where the injection of an antigenic composition (the vaccine) provokes an immune response against the antigen, i.e. in case of cancer against tumor specific antigens. Typical cancer vaccines are dendritic cell therapy/vaccines (DC vaccines), adoptive T-cell therapy, peptidic, DNA or mRNA vaccines or oncolytic viruses. In a preferred embodiment, DC vaccines are used.

"DC vaccines" refers to DCs for therapeutic use that may be administered, being prepared without an antigen source or with an antigen source. Preferably, the DCs have been prepared with an antigen source selected from tumor associated peptide(s), whole antigens from DNA or RNA, whole antigen-protein, idiotype protein, tumor lysate/whole tumor cells or viral vector-delivered whole antigen.

"Tregs" refers to T-cells, which are positive for CD4, CD25, and the Foxp3 transcription factor, which suppress the function and proliferation of tumor-specific CD4⁺ and CD8⁺ T cells and NK cells.

"Myeloid-derived suppressor cells" or "MDSCs" are reported to induce Tregs (Malek et al., 2016) and limit effector T-cell proliferation by means of the production of various immunosuppressive molecules. More specifically, MDSCs consist of two large groups of cells termed "polymorphonuclear" (PMN-MDSCs) - alternatively also "granulocytic" (G-MDSCs), which are phenotypically and morphologically similar to neutrophils, and "monocytic" (M-MDSCs), phenotypically and morphologically similar to monocytes. Further, early-stage MDSCs (eMDSCs) are existing (Gabrilovich, 2017). Within this application, the term MDSC when used solely is to be understood to encompass M-MDSC and PMN-MDSC. In humans PMN-MDSCs are defined as CD11b⁺CD14⁻CD15⁺ or CD11b⁺CD14⁻CD66b⁺, and M-MDSCs as CD11b⁺CD14⁺HLA-DR^{-/lo}CD15⁻. M-MDSCs can be separated from monocytes based on the expression of the MHC class II molecule HLA-DR. PMN-MDSCs can be separated from neutrophils for example by gradient centrifugation using a standard Ficoll gradient. PMN-MDSCs are enriched in the low-density fraction (PBMCs), whereas neutrophils are high density cells (Dumitru et al., 2012). Recently it was found that the lectin-type oxidized LDL receptor 1 (LOX-1) can be used as a marker of PMN-MDSCs (Condamine et al., 2016).

"MDSC-inhibiting agent" refers to a molecule that decreases, blocks and/or inhibits MDSCs, which suppresses proliferation or differentiation of MDSCs, e.g. all-trans-retinoic acid (ATRA) inhibits differentiation of MDSCs. An MDSC-inhibiting agent blocks or inhibits differentiation/maturation of MDSCs, blocks or inhibits migration of MDSCs or induces depletion of MDSCs/apoptosis of MDSCs, or inhibits expansion of MDSCs. Alternatively, MDSC-inhibiting agents may also be grouped into molecules which deactivate MDSCs, which inhibit the differentiation of MDSCs into mature cells, which block the development of MDSCs or which deplete MDSCs (Wesolowski et al., 2013). In this context, molecule preferably refers to a low molecular weight molecule with a molecular weight ≤ 2500 Dalton, especially ≤ 900 Daltons, and/or a biopharmaceutical macromolecule, preferably a recombinant therapeutic protein.

"Healthy individual" refers to an individual with no history of malignant disease. Similarly, "healthy tissue" refers to tissue with no sign of malignant disease of the same organ from which the tumor to be treated is derived, e.g. lung tissue.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Technical terms are used by their common sense. If a specific meaning is conveyed to certain terms, definitions of terms will be given in the following in the context of which the terms are used.

### Description of the Invention

The present invention relates to a composition comprising a dendritic cell therapy/vaccine (DC vaccine) for use in treating or delaying progression of squamous cell carcinoma (SCC) of the lungs in an individual, wherein the treatment comprises
administration of the composition in combination with a second composition comprising
a myeloid-derived suppressor cell (MDSC)-inhibiting agent and an optional pharmaceutically acceptable carrier,
wherein the DC vaccine has been prepared with an antigen source selected from tumor associated peptide(s), whole antigens from DNA or RNA, whole antigen-protein, idiotype protein, tumor lysate/whole tumor cells or viral vector-delivered whole antigen,
wherein the MDSC-inhibiting agent blocks or inhibits differentiation/maturation of MDSCs, blocks or inhibits migration of MDSCs or induces depletion of MDSCs/apoptosis of MDSCs, or inhibits expansion of MDSCs, or inhibits MDSC effector functions, and
wherein the MDSC-inhibiting agent that blocks or inhibits differentiation/maturation of MDSCs is selected from all-trans-retinoic acid, Curcumin derivatives; wherein the inhibitor of migration of MDSCs is selected from Zolendronic acid, anti-glycan antibodies and CSF-1R inhibitors; wherein the inducer of depletion or apoptosis of MDSCs is selected from tyrosine kinase inhibitors, preferably Sunitinib, anti-Gr1 antibodies, IL4Rα aptamer, Gemcitabine, Cisplatin, Paclitaxel, 17-Dimethylaminoethylamino-17-demethoxygeldanamycin (17-DMAG) or 5-fluorouracil (5-FU); wherein the inhibitor of expansion of MDSCs is selected from Bevacizumab, Celecoxib and Pimozide; and wherein the inhibitor of MDSC effector functions is an inducer of oxidative stress and is selected from N-hydroxyl-L-Arginine (NOHA), Nitroaspirin, N-acetyl cysteine (NAC), CpG oligodeoxy-nucleotides, Bardoxolone methyl (CDDO-Me), Withaferin A or Stattic.

The present invention also relates to a composition comprising a dendritic cell therapy/vaccine (DC vaccine) for use in treating or delaying progression of non-small cell lung cancer (NSCLC) in an individual, wherein the treatment comprises
administration of the composition in combination with a second composition comprising
a myeloid-derived suppressor cell (MDSC)-inhibiting agent and an optional pharmaceutically acceptable carrier
wherein the individual is characterized by a immunosuppressive tumor microenvironment caused by the presence of MDSCs,
wherein the DC vaccine has been prepared with an antigen source selected from tumor associated peptide(s), whole antigens from DNA or RNA, whole antigen-protein, idiotype protein, tumor lysate/whole tumor cells or viral vector-delivered whole antigen,
wherein the individual with a immunosuppressive tumor microenvironment is further characterized in that
   - the fraction of living cells in peripheral blood mononuclear cells which are monocytic MDSCs (M-MDSCs) is at least 0.08%, preferably at least 0.1%,
   - the fraction of living cells in tumor tissue which are M-MDSCs, is at least 0.005%, preferably at least 0.008%,
   - the level of LOX-1 in serum or plasma is above 75 pg/ml, preferentially above 100 pg/ml.
   - the percentage of LOX-1-expressing PMN-MDSC among all PMN-MDSC is at least 5%, preferentially 10%.
   - the fraction of living cells in tumor tissue which are PMN-MDSCs is at least 2%, preferably at least 2.5%,
   - the fraction of living Treg cells in peripheral blood mononuclear cells is at most 1.8%, preferably at most 1.5%,
   - the fraction of living Treg cells in tumor tissue is at most 10% of CD4+ cells, preferably at most 9% of CD4+ cells,
   - the fraction of CD3ζ among CD4+ T cells in peripheral blood mononuclear cells is reduced compared to control cells of healthy individuals by a factor of at least 0.9, preferably at least by a factor of 0.8,)
   - the fraction of CD3ζ among CD8+ T cells in peripheral blood mononuclear cells is reduced compared to control cells of healthy individuals by a factor of at least 0.75, preferably at least by a factor of 0.65, and/or
   - the relative expression of Arginase 1 in peripheral blood mononuclear cells of patients compared to control peripheral blood mononuclear cells of healthy individuals is increased at least by a factor of 2.5,
wherein the MDSC-inhibiting agent blocks or inhibits differentiation/maturation of MDSCs, blocks or inhibits migration of MDSCs or induces depletion of MDSCs/apoptosis of MDSCs, or inhibits expansion of MDSCs, or inhibits MDSC effector functions, and
wherein the MDSC-inhibiting agent that blocks or inhibits differentiation/maturation of MDSCs is selected from all-trans-retinoic acid, Curcumin derivatives; wherein the inhibitor of migration of MDSCs is selected from Zolendronic acid, anti-glycan antibodies and CSF-1R inhibitors; wherein the inducer of depletion or apoptosis of MDSCs is selected from tyrosine kinase inhibitors, preferably Sunitinib, anti-Gr1 antibodies, IL4Rα aptamer, Gemcitabine, Cisplatin, Paclitaxel, 17-Dimethylaminoethylamino-17-demethoxygeldanamycin (17-DMAG) or 5-fluorouracil (5-FU); wherein the inhibitor of expansion of MDSCs is selected from Bevacizumab, Celecoxib and Pimozide; and wherein the inhibitor of MDSC effector functions is an inducer of oxidative stress and is selected from N-hydroxyl-L-Arginine (NOHA), Nitroaspirin, N-acetyl cysteine (NAC), CpG oligodeoxy-nucleotides, Bardoxolone methyl (CDDO-Me), Withaferin A or Stattic.

Cancer heterogeneity, a hallmark enabling clonal survival and therapy resistance, is shaped by active immune responses. Antigen-specific T cells can control cancer as shown clinically by immunotherapeutics such as adoptive T-cell transfer and checkpoint blockade. However, cancer immunotherapies still have a low success rate and a detailed understanding of who will benefit and why is required (Harrop, 2013).

In the study underlying the invention, immune cell infiltration, T cell responses and cytokine production in primary tumors (Tu) and non-tumoral lung tissue (NTu) from initially 38 adenocarcinomas (AC) and 35 squamous cell carcinomas (SCC) of non-small cell lung cancer (NSCLC) patients undergoing neoadjuvant surgery and blood samples from 13 AC and 12 SCC patients and 10 age-matched controls/benign patients (see Table 1) were compared. It is to be understood that the term "PBMC" refers to respective PBMCs that are isolated from blood samples by methods commonly known to the skilled person, unless it is expressly indicated that the PBMCs have been isolated from other sources. In an effort to complete the study, the sample collection was expanded and reached 43 AC and 39 SCC tumor tissue samples, 32 AC, 30 SCC and 17 healthy age-matched control blood samples as well as 57 AC, 52 SCC and 41 healthy age-matched control plasma blood samples (see Table 2). Similarly, populations of myeloid-derived suppressor cells (MDSC) and CD4⁺CD25⁺Foxp3⁺ T regulatory cells in PBMCs of NSCLC patients and healthy aged-matched donors were compared. In both tumor subtypes significantly higher infiltration of various immune cell populations compared to non-tumoral tissue was observed. In tumor samples, the study confirmed earlier published data (Black et al., 2013) showing that there is higher CD4⁺CD25⁺Foxp3⁺ Tregs infiltration in AC than in SCC.

However, surprisingly in SCC patients the populations of CD15⁻CD14⁺CD33^{hi}HLA-DR^{-/lo} M-MDSC from blood and tumor samples and CD11b⁺CD14⁻CD15⁺ PMN-MDSC from tumor samples were significantly enhanced compared to AC patients and the controls. Additionally down-regulation of CD3ζ in CD4⁺ and CD8⁺ T cells and up-regulation of ARG1 expression in PBMC of SCC patients was observed. The downregulation of CD3ζ in both CD4⁺ and CD8⁺ T cells negatively correlated with the presence of PMN-MDSC. Functional suppression tests suggest that M-MDSC and PMN-MDSC play the main inhibitory role over Tregs in SCC patients, but not in AC.

Further, the study aimed to analyze the infiltration of immune cells in primary NSCLC tumors and non-tumoral tissues, to analyze intra-tumoral IFN-γ producing CD4⁺ and CD8⁺ T cells and cytokine production after stimulation. The presence of MDSC and CD4⁺CD25⁺Foxp3⁺ Tregs in the tumor and peripheral blood of NSCLC patients and age-matched healthy controls was also analyzed.

Based on the comparative studies of SCC and AC it was found that the immune cell infiltration is higher in tumors than in non-tumoral tissue in both subtypes. There was no difference in the infiltration of immune cells except for conventional dendritic cells (DC) (CD11c⁺HLA⁻DR^{hi}). These DC were significantly decreased in SCC in comparison to AC, which suggests lower antigen presentation capacity in SCC tumors.

Surprisingly, when the peripheral blood mononuclear cell population was analyzed, monocytic CD15⁻CD14⁺CD33^{hi}HLA-DR^{-/lo} MDSC population, down-regulation of CD3ζ in CD4⁺ and CD8⁺ T cells and up-regulation of ARG1 in PBMC was only observed in blood of SCC patients. Both AC and SCC MDSC inhibit T cell proliferation, but a stronger inhibition of IL-2 and IFN-γ in T-cells was only observed for SCC. Therefore, MDSC form SCC had a higher suppressive activity. Furthermore, when tumor-infiltrating MDSCs were analyzed, CD11b⁺CD14⁻CD15⁺ PMN-MDSCs and CD14⁺CD33^{hi}HLA-DR^{-/lo} M-MDSC populations were higher in SCC tumors than in AC tumors. CD4⁺CD25⁺Foxp3⁺ CD127^{low} Tregs were enhanced only in frozen blood samples of AC patients and negatively correlated with CD15⁻CD14⁺CD33^{hi}HLA-DR^{-/lo} MDSC population, suggesting differences in mechanisms of the systemic immunosuppression between AC and SCC histotypes in NSCLC. The same results were observed when fresh blood samples were analyzed. Tregs negatively correlated with CD11b⁺CD14⁻CD15⁺ PMN-MDSCs in AC but not in SCC. This could not been detected in frozen samples due to very high rate of PMN-MDSCs decay after cryopreservation. No difference between AC and SCC was observed when the amount of CD11b⁺CD14⁻CD15⁺ PMN-MDSCs was analyzed in patient's blood, whether fresh or frozen based on the phenotypic characterization. When tumor samples were analyzed, Tregs were detected in higher numbers in tumors than in non-tumoral tissue in both histological subtypes of NSCLC. However infiltration of Tregs was higher in AC than SCC tumors.

In summary, in SCC the immunosuppressive tumor microenvironment has been shown to be dominated by MDSCs, whereas MDSCs have only a minor role in most of the cancer patients suffering from AC. As MDSCs have been shown to have a strong immunosuppressive activity in the tumor microenvironment (Umansky et al., 2016), a solution to harness the host immune system in order to fight SCC or NSCLC with an MDSC phenotype can be to combine immunotherapeutics such as dendritic cell vaccines with inhibitors of MDSCs.

Therefore, in a first aspect, the thepresent invention relates to a composition comprising a dendritic cell therapy/vaccine (DC vaccine) for use in treating or delaying progression of squamous cell carcinoma (SCC) in an individual suffering from SCC. The treatment comprises administration of the composition in combination with a second composition comprising a myeloid-derived suppressor cell (MDSC)-inhibiting agent or an inhibitor of MDSC effector functions and an optional pharmaceutically acceptable carrier.

Based on the surprising finding that MDSCs are the dominating immunosuppressive cells in SCC, it follows that their inhibition or the inhibition of their effector functions results in significantly reduced suppression of the immune system in the tumor microenvironment and accordingly makes a combined immunotherapy more effective. This may be an important way to overcome the serious challenges of immunotherapy in general and to overcome low clinical efficacy despite the advances in identifying tumor-associated antigens recognized by cytotoxic T-Cells (CTLs) (Ramakrishnan et al., 2010).

Such individual suffering from SCC may be further characterized in that the fraction of living cells in peripheral blood mononuclear cells (PBMCs), which are CD15⁻CD14⁺CD33^{hi}HLA⁻DR^{-/lo} M-MDSC, is at least 0.08%, preferably at least 0.1%. The % values refer to % of total number of living cells of the PBMCs. These percentages of M-MDSC correspond to measurements obtained from previously cryopreserved PBMC samples by cell sorting as described in example 7. When the fraction of living cells in PBMC is measured in fresh non-cryopreserved samples, individuals may be characterized in that the fraction of living cells PBMCs, which are M-MDSC, is at least 0.25%, and preferably at least 0.35%. PBMCs may be collected from patient's blood and isolated by Ficoll-Pague gradient centrifugation, or other techniques know in the art. Dead cells may be discriminated from living cells by cytometry using DAPI stain or LIVE/DEAD^{®} Fixable Aqua Dead Cell Stain or any other techniques known in the art. M-MDSCs may be measured for example by flow cytometry using specific gating parameters, involving anti-CD14 antibodies, anti-CD33 antibodies and anti-HLA-DR antibodies. In a particularly preferred embodiment, the fraction of M-MDSCs is determined as described in the method of example 7.

The individuals suffering from SCC may further be characterized in that the fraction of living cells in tumor tissue which are M-MDSCs, is at least 0.005%, preferably at least 0.008%. In a preferred embodiment, the tumor tissue is fresh tumor tissue, i.e. tumor tissue that has not been frozen. In another embodiment, the tumor tissue sample was frozen.

Another means to characterize individuals suffering from SCC is that the LOX-1 in serum or plasma levels are at least 75 pg/ml, preferably at least 100 pg/ml. Serum or plasma can be obtained from patient's blood by techniques know in the art. LOX-1 stands for lectin-type oxidized low-density lipoprotein receptor 1 (LOX-1), OLR1, CLEC8A, LOX1, LOXIN, SCARE1, SLOX1 or oxidized low density lipoprotein receptor 1 (Ox-LDL receptor 1). LOX-1 has been identified as a major receptor for oxidized low-density lipoprotein (ox-LDL) in endothelial cells, monocytes, platelets, cardiomyocytes, and vascular smooth muscle cells. Its expression is minimal under physiological conditions but can be induced under pathological conditions (Lu et al., 2011a). It can be cleaved and released in a soluble form into the circulation (Biocca et al., 2013). Levels of soluble LOX-1 in blood or plasma can be measured by techniques known in the art, for example by ELISA tests.

Another means to characterize individuals suffering from SCC is that at least 5%, preferentially at least 10% of CD15⁺ peripheral blood mononuclear cells are LOX-1⁺ (LOX-1-expressing PMN-MDSC). The percentage of LOX-1 positive cells is measured as described in the methods of example 7 from the MDSC in peripheral blood positive for CD15.

The individuals suffering from SCC may also be characterized in that the fraction of living cells in tumor tissue, which are PMN-MDSCs, is at least 2%, preferably at least 2.5%. The fraction of PMN-MDSCs was determined as described in example 7. It is understood, when measured by flow cytometry, that counted PMN-MDSCs may also contain a fraction of neutrophils, recognized by the same antibodies. Neutrophils can be separated from PMN-MDCS prior to flow cytometry measurements by Ficoll gradient. PMN-MDSCs are enriched in the low-density fraction, whereas neutrophils are high density cells.

In another aspect, such individuals suffering from SCC may be characterized in that the fraction of living PBMC, which are Treg cells, is at most 1.8%, preferably at most 1.5%. The % values refer to % of total number of living cells of the PBMCs. These percentages of Treg cells correspond to measurements obtained from previously cryopreserved PBMC samples. When the fraction of living cells in PBMC is measured in fresh non-cryopreserved samples, individuals may be characterized in that the fraction of living PBMC, which are Treg cells, is at most 1%, preferably at most 0.8%. In a particularly preferred embodiment, the fraction of Tregs is determined as described in the method of example 7.

The fraction of living Treg cells in tumor tissue may also be used to characterize individuals with a MDSC-dominated tumor environment. In this case, the fraction of living PBMC which are Treg cells in tumor tissue is at most 10% of CD4⁺ cells, preferably at most 9% of CD4+ cells. The inventors conclude that SCC patients generally have an immunosuppressive tumor environment and that if this is not due to high Treg cells in tumor tissue, this in turn is based on high MDSCs.

In yet another aspect, such individuals suffering from SCC may be characterized in that the fraction of CD3ζ among CD4⁺ T cells in PBMC is reduced compared to control CD4⁺ T cells of healthy individuals by a factor of at least 0.9, preferably at least by a factor of 0.8. These fractions of CD3ζ correspond to measurements obtained from previously cryopreserved PBMC samples. When the fraction of living cells in PBMC is measured in fresh non-cryopreserved samples, individuals may be characterized in that the fraction of CD3ζ among CD4⁺ T cells in PBMC is reduced compared to control CD4⁺ T cells of healthy individuals by a factor of at least 0.7, preferably at least by a factor of 0.6. The fraction of CD3ζ among CD4⁺ T cells can be for example measured by immune-staining of isolated CD4⁺ T cells with an anti-CD3ζ antibody. Decrease in CD3ζ expression on T cells has been previously correlated with increased MDSC suppressive activity, and can be explained by shortage of L-arginine due to increased ARG1 activity of MDSCs (Gabrilovich and Nagaraj, 2009).

In another embodiment, such individuals suffering from SCC may be characterized in that the fraction of CD3ζ among CD8⁺ T cells PBMC is reduced compared to control CD8⁺ T cells of healthy individuals by a factor of at least 0.75, preferably at least by a factor of 0.65. These fractions of CD3ζ correspond to measurements obtained from previously cryopreserved PBMC samples. When the fraction of living cells in PBMC is measured in fresh non-cryopreserved samples, individuals may be characterized in that the fraction of CD3ζ among CD8⁺ T cells in PBMC is reduced compared to control CD8⁺ T cells of healthy individuals by a factor of at least 0.7, preferably by a factor of at least 0.5.

In yet another embodiment, individuals suffering from SCC are characterized in that the relative expression of ARG1 of PBMC, compared to PBMC cells of healthy individuals is increased at least by a factor of 2.5. Relative expression of ARG1 of PBMC can be measured by quantitative PCR. In a preferred embodiment, the level is determined by quantitative PCR as described in Example 7.

In a third aspect the present invention relates to a composition comprising a dendritic cell therapy/vaccine (DC vaccine) for use in treating or delaying progression of non-small cell lung cancer (NSCLC) in an individual, wherein the treatment comprises administration of the composition in combination with a second composition comprising a myeloid-derived suppressor cell (MDSC)-inhibiting agent or an inhibitor of MDSC effector functions and an optional pharmaceutically acceptable carrier, wherein the individual is further characterized by an immunosuppressive tumor microenvironment caused by the presence of MDSCs. In the context of the present invention, an immunosuppressive tumor microenvironment caused by the presence of MDSCs implicates the suppression of different cells of the immune system, mainly T cells, by MDSCs. The main factors involved in MDSC-mediated immune suppression include increased expression of ARG1, increased expression of iNOS, increased production of TGF beta and IL10 by MDSC, increased activity of COX2 in MDSC, increased indoleamine 2,3-dioxygenase (IDO) expression by MDSC or tumor cells, sequestration of cysteine by MDSC, decrease of L-selectin expression by T cells, and many others (Bronte et al., 2016, Table 3. Any difference in these factors, compared to healthy tissue, may be used to characterize an immunosuppressive tumor microenvironment. It is believed that M-MDSCs suppress T-cell responses by utilizing mechanisms associated with production of NO and cytokines (reviewed in Gabrilovich et al., 2012). On the other hand, PMN-MDSCs suppress immune responses primarily in an antigen-specific manner by for example inducing antigen-specific T-cell tolerance (Koehn et al., 2015, Marigo et al., 2010).

Whereas it was generally observed in the context of this invention that SCC patients have a significantly enhanced presence of MDSCs, there was also a number of AC patients having similar levels of MDSCs. Accordingly, it follows that any NSCLC patient having an immunosuppressive tumor microenvironment caused by the presence of MDSCs will profit from the combined treatment with an immunogenic composition and an MDSC-inhibiting agent or an inhibitor of MDSC effector functions.

In another aspect, the present invention relates to a composition comprising a dendritic cell therapy/vaccine (DC vaccine) for use in treating or delaying progression of non-small cell lung cancer (NSCLC) in an individual, wherein the treatment comprises administration of the composition in combination with a second composition comprising a myeloid-derived suppressor cell (MDSC)-inhibiting agent or an inhibitor of MDSC effector functions and an optional pharmaceutically acceptable carrier, wherein the individual with an immunosuppressive tumor microenvironment caused by the presence of MDSCs is characterized in that the fraction of living cells in PBMCs which are M-MDSCs is at least 0.08%, preferably at least 0.1%, These percentages of M-MDSC correspond to measurements obtained from previously cryopreserved PBMC samples. When the fraction of living cells in PBMC is measured in fresh non-cryopreserved samples, individuals may be characterized in that the fraction of living cells PBMCs, which are M-MDSC, is at least 0.25%, preferably 0.35%.

The individuals with an immunosuppressive tumor microenvironment caused by the presence of MDSCs can further be characterized in that the fraction of living cells in tumor tissue which are M-MDSCs, is at least 0.005%, preferably at least 0.008%. In a preferred embodiment, the tumor tissue is fresh tumor tissue, i.e. tumor tissue that has not been frozen, due to the sensitivity of MDSCs to freeze/thaw.

Another means to characterize individuals with an immunosuppressive tumor microenvironment caused by the presence of MDSCs is that the LOX-1 in serum or plasma levels are at least 75 pg/ml, preferably at least 100 pg/ml. Serum or plasma can be obtained from patient's blood by techniques know in the art.

Another means to characterize individuals with an immunosuppressive tumor microenvironment caused by the presence of MDSCs is that at least 5%, preferentially at least 10% of CD15⁺ peripheral blood mononuclear cells are LOX-1⁺ (LOX-1-expressing PMN-MDSC). The percentage of LOX-1 positive cells is measured as described in the methods of example 7, from the MDSC in peripheral blood positive for CD15.

Individuals with an immunosuppressive tumor microenvironment caused by the presence of MDSCs can also be characterized in that the fraction of living cells in tumor tissue, which are PMN-MDSCs, is at least 2%, preferably at least 2.5%. The fraction of PMN-MDSCs was determined as described in example 7. It is understood, when measured by flow cytometry, that counted PMN-MDSCs may also contain a fraction of neutrophils, recognized by the same antibodies. Neutrophils can be separated from PMN-MDCS prior to flow cytometry measurements by Ficoll gradient. PMN-MDSCs are enriched in the low-density fraction, whereas neutrophils are high density cells.

Individuals with an immunosuppressive tumor microenvironment caused by the presence of MDSCs may be characterized in that the fraction of living Treg cells in peripheral blood mononuclear cells is at most 1.8%, preferably at most 1.5%. These percentages of Treg cells correspond to measurements obtained from previously cryopreserved PBMC samples. When the fraction of living cells in PBMC is measured in fresh non-cryopreserved samples, individuals may be characterized in that the fraction of living PBMC which are Treg cells is at most 1%, preferably at most 0.8%.

The fraction of living Treg cells in tumor tissue can also be used to characterize individuals with an immunosuppressive tumor microenvironment. In this case, the fraction of living PBMC which are Treg cells in tumor tissue is at most 10% of CD4⁺ cells, preferably at most 9% of CD4⁺ cells. The inventors conclude that NSCLC patients generally have an immunosuppressive tumor environment and that if this is not due to high Treg cells in tumor tissue, this in turn is based on high MDSCs.

A decrease in CD3ζ is an indirect measure due to an elevated ARG1 activity that causes T-cell suppression through the depletion of arginine (Gabrilovich and Nagaraj, 2009), for both CD4⁺ and CD8⁺T cells in PBMC.

Accordingly, another means to characterize individuals with an immunosuppressive tumor microenvironment caused by the presence of MDSCs is that the fraction of CD3ζ among CD4⁺ T cells in PBMC is reduced compared to control cells of healthy individuals by a factor of at least 0.9, preferably at least by a factor of 0.8. These fractions of CD3ζ correspond to measurements obtained from previously cryopreserved PBMC samples. When the fraction of living cells in PBMC is measured in fresh non-cryopreserved samples, individuals may be characterized in that the fraction of CD3ζ among CD4⁺ T cells in PBMC is reduced compared to control cells of healthy individuals by a factor of at least 0.7, preferably at least by a factor of 0.6. The fraction of CD3ζ among CD4⁺ T cells can be for example measured by immune-staining of isolated CD4⁺ T with an anti-CD3ζ antibody.

And further, another means to characterize individuals with an immunosuppressive tumor microenvironment caused by the presence of MDSCs is that the fraction of CD3ζ among CD8⁺ T cells PBMC is reduced compared to control cells of healthy individuals by a factor of at least 0.75, preferably at least by a factor of 0.65. These fractions of CD3ζ correspond to measurements obtained from previously cryopreserved PBMC samples. When the fraction of living cells in PBMC is measured in fresh non-cryopreserved samples, individuals may be characterized in that the fraction of CD3ζ among CD8⁺ T cells in PBMC is reduced compared to control cells of healthy individuals by a factor of at least 0.7, preferably at least by a factor of 0.5.

Individuals with an immunosuppressive tumor microenvironment caused by the presence of MDSCs can also be characterized in the relative expression of ARG1 in PBMC, compared to PBMC of healthy individuals is increased at least by a factor of 2.5. Relative expression of ARG1 in PBMC can be measured by quantitative PCR.

In an aspect of the present invention, M-MDSCs are characterized as having a CD14⁺CD15⁻CD33^{hi}HLA-DR^{-/lo} phenotype and PMN-MDSCs as having a suppressive CD14⁻CD15⁺CD11b⁺ phenotype, preferably expressing LOX-1. Flow cytometry or other techniques known in the art can be used to phenotypically characterize these cells. The phenotypical characterization of PMN-MDSCs as CD14⁻CD15⁺CD11b⁺ may also apply to neutrophils. However, neutrophil contaminations in the tumor patients of this study are considered negligible. On the other hand, neutrophils could be separated from PMN-MDCS prior to flow cytometry measurements by Ficoll gradient (Scapini and Cassatella, 2014). PMN-MDSCs are enriched in the low-density fraction (PBMCs), whereas neutrophils are high density cells. Furthermore, neutrophils do not possess the suppressive abilities, typically attributed to PMN-MDSCs. Suppression can be shown for example by inhibition of anti-CD3/CD28 (or polyhydroxyalkanoates) induced T-cell proliferation or IL-2 and IFN-γ production. ³H-thymidine incorporation or CFSE dilution can be used to measure T-cell proliferation and ELISPOT or intracellular staining to measure IFN-γ production.

In another aspect, the composition is for use in treating or delaying progression of SCC of the lungs. Squamous cell carcinoma of the lungs is one form of non-small cell lung cancer. SCC begins in the tissue that lines the air passages in the lungs. It is also known as epidermoid carcinoma. Most SCCs of the lungs are located centrally, usually in the larger bronchi that join the trachea to the lung. SCCs are linked more strongly with smoking than other forms of non-small cell lung cancers and are more common in men than in women. They tend to be slow-growing, and due to their location are often found earlier than other forms of lung cancer. Common symptoms of lung cancer include a persistent cough, coughing up blood, and wheezing. Since SCC tends to be located near the large airways, they often cause symptoms earlier than other forms of lung cancer. Obstruction of the airway can lead to infections such as pneumonia, or collapse of part of a lung (atelectasis). SCC is the most common cause of the Pancoast syndrome or superior sulcus syndrome. Pancoast syndrome is caused by lung cancers that begin near the top of the lungs and invade structures nearby. Symptoms often include shoulder pain that radiates down the inside of the arm, weakness or prickly sensations in the hands, flushing or sweating on one side of the face, and a droopy eyelid (Horner's syndrome). Individuals with SCC are also more likely to experience a hypercalcemia which can result in muscle weakness and cramps. Hypercalcemia is one of the symptoms of paraneoplastic syndrome and is caused by a tumor secreting a hormone-like substance that raises the calcium level in the blood.

In another aspect of the present invention, the composition is for use in treating an individual which has been diagnosed with SCC. Diagnosis of SCC is well known in the art (Travis et al., 2015). SCC of the lungs is often first suspected when abnormalities are seen on an X-ray. Further evaluation may include: chest CT scan, sputum cytology, bronchoscopy, PET Scan, endobronchial ultrasound. Depending upon the results, a lung biopsy may be obtained to confirm the diagnosis (Travis et al., 2013). SCC of the lungs is broken down into 4 stages:
Stage I: the cancer is localized within the lung and has not spread to any lymph nodes
Stage II: the cancer has spread to lymph nodes or the lining of the lungs, or is in a certain area of the main bronchus
Stage III: the cancer has spread to tissue near the lungs
Stage IV: the cancer has spread (metastasized) to another part of the body.

In another aspect of the invention, the composition for use in treating or delaying progression of SCC of the lungs may be administered in combination with the standard treatment of care (which presently does not distinguish between SCC and AC), preferably carboplatin plus paclitaxel for the treatment of stage IV NSCLC, pemetrexed plus carboplatin for the treatment of stage IV EGFR wild type NSCLC, gemcitabine plus cisplatin or pemetrexed plus cisplatin for the treatment of stage IIIB to IV, EGFR wild type NSCLC or vinorelbine plus carboplatin for the treatment of stage IIIA-IV NSCLC.

The invention relates to an immunogenic composition in combination with an MDSC-inhibiting agent or an inhibitor of MDSC effector functions, wherein the immunogenic composition is a dendritic cell therapy/vaccines (DC vaccine). Different techniques of adoptive T-cell therapies have been described in Houot R et al. (2015).

Different techniques of peptidic vaccines have been described in Xiao YF et al. (2015). Different techniques of DNA vaccines have been described in Yang B et al. (2014). Different techniques of mRNA vaccines have been described in McNamara MA et al. (2015). Different techniques of oncolytic virus vaccines have been described in Aitken AS et al. (Aitken et al., 2017). Different techniques of dendritic cell vaccines have been described in Turnis and Rooney, (2010).

In a preferred embodiment, the DC vaccine is prepared with an antigen source selected from tumor associated peptide(s), whole antigens from DNA or RNA, whole antigen-protein, idiotype protein, tumor lysate/whole tumor cells or viral vector-delivered whole antigen, as exemplified in Table 1 of Tumis and Rooney, (2010).

In an especially preferred embodiment such whole tumor cells, which are used for loading onto immature DCs, have been prepared by high hydrostatic pressure (HHP), as described in WO 2013/004708 and WO 2015/097037. Treating whole tumor cells with HHP allows inducing immunogenic cell death (ICD), a specific type of apoptosis. Tumor cells dying of ICD will express very potent "eat-me" signals, such as calreticulin, HSP70 and HSP90. These "eat-me" signals will trigger efficient phagocytosis of the apoptotic tumor cells by immature DC and efficient presentation of tumor antigen by the matured DC. Preferred conditions for generating a potent NSCLC or SCC DC vaccine using HHP is subjecting NCI-H520 or NCI-H522 lung cancer cell lines, combined or single, to a pressure of 250 MPa for 10 min, and loading immature DCs with the resulting cells undergoing immunogenic cell death.

In a preferred embodiment, the MDSC-inhibiting agent blocks or inhibits differentiation/maturation of MDSCs, blocks or inhibits migration of MDSCs or induces depletion of MDSCs/apoptosis of MDSCs, or inhibits expansion of MDSCs. Preclinical and clinical compounds targeting MDSCs are listed in Table 1 of Draghiciu et al. (2015), in Table 2 of Ko and Kim (2016) and in Table 1 and Box 2 of Talmadge and Gabrilovich (2013).

Preferred examples of inhibitors of differentiation/maturation of MDSCs are all-trans-retinoic acid (ATRA) and Curcumin derivatives. ATRA may be administrated a few days prior to vaccination, at 150 mg/m². Preferred examples of inhibitors of migration of MDSCs are Zolendronic acid, anti-glycan antibodies and CSF-1R inhibitors. Preferred examples of inducers of depletion or apoptosis of MDSCs are tyrosine kinase inhibitors, preferably Sunitinib, anti-Gr1 antibodies, IL4Rα aptamer, Gemcitabine, Cisplatin, Paclitaxel, 17-Dimethylaminoethylamino-17-demethoxygeldanamycin (17-DMAG) and 5-fluorouracil (5-FU). Preferred examples of inhibitors of expansion of MDSCs are Bevacizumab, Celecoxib and Pimozide. Preferred examples of inhibitors of MDSC effector functions are inducers of oxidative stress, preferably N-hydroxyl-L-Arginine (NOHA), Nitroaspirin, N-acetyl cysteine (NAC), CpG oligodeoxy-nucleotides, Bardoxolone methyl (CDDO-Me), Withaferin A and Stattic.

Especially preferred is the combination with ATRA, paclitaxel, gemcitabine or cisplatin.

### Description of the Figures

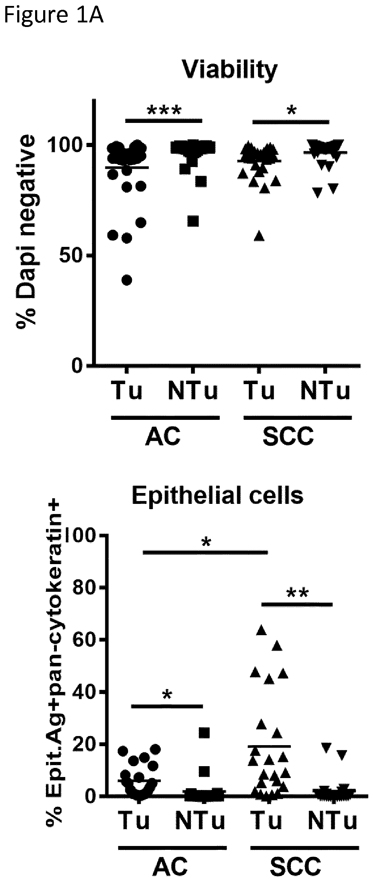

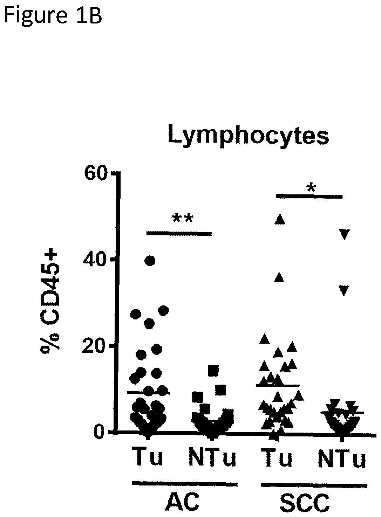

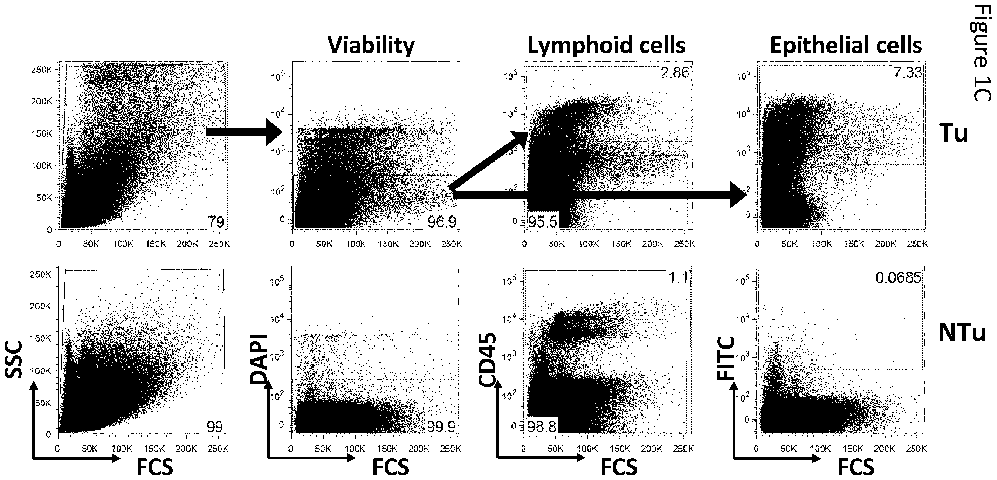

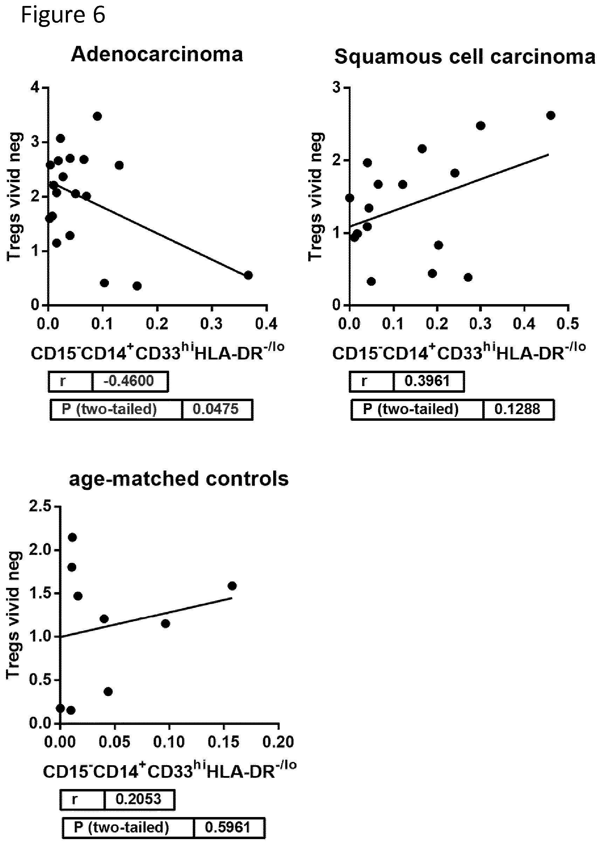

Figure 1: Viability and origin of cells present in the tumors and healthy tissue.
   (A) Viability of all isolated cells isolated from tumor (Tu) and non-tumor healthy lung tissue (NTu) was measured by DAPI staining followed by flow cytometry top panel). The percentage of epithelial cells in AC or SCC tumor (Tu) and non-tumor healthy lung tissue (NTu) was measured by flow cytometry after immune-staining for cytokeratin bottom panel).
   (B) The percentage of lymphocytes in tumor (Tu) and non-tumor healthy lung tissue (NTu) was measured by flow cytometry after immune-staining for CD45.
   (C) Gating strategy for flow cytometry.

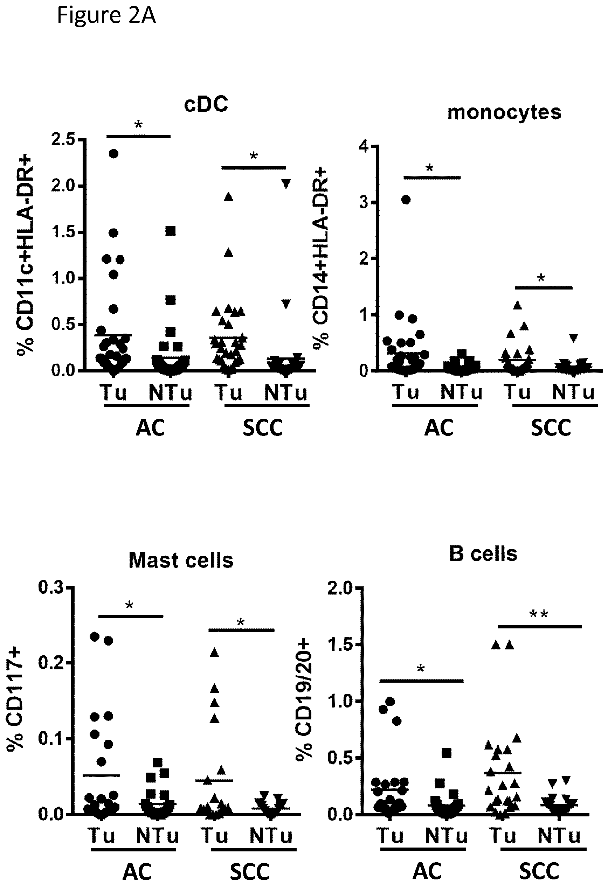

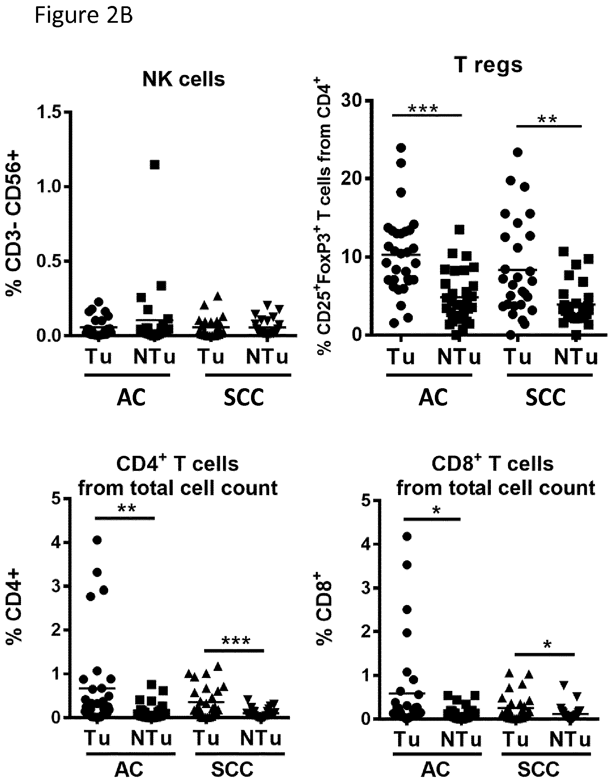

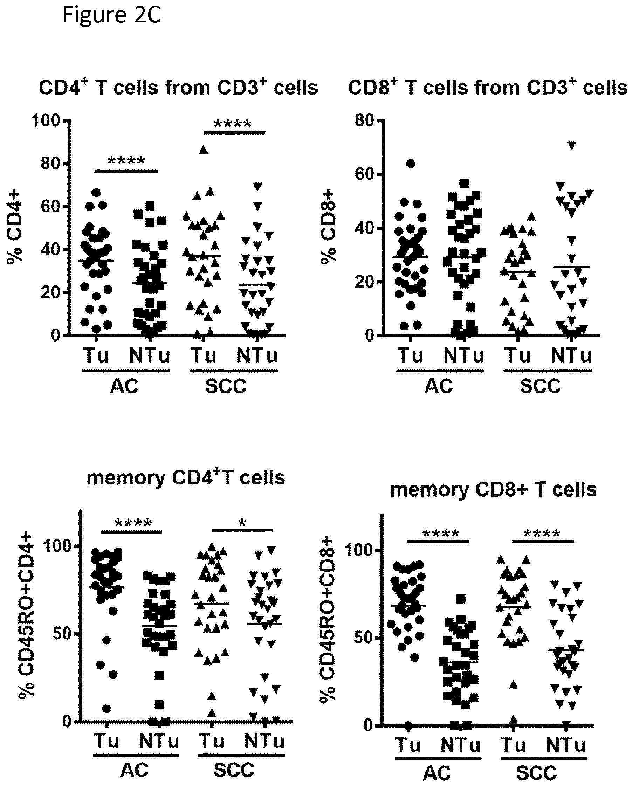

Figure 2: The immune cell infiltration in NSCLC tumors and non-tumoral tissue in adenocarcinoma (AC) and squamous cell carcinoma (SCC).
   (A) The percentage of cDC (CD11c+ HLA-DR+, top left panel), monocytes (CD14+ HLA-DR+, top right panel), mast cells (CD117+, bottom left panel) and B cells (CD19/20+, bottom right panel) from live cells in AC or SCC tumor (Tu) and non-tumor healthy lung tissue (NTu) was estimated by flow cytometry.
   (B) The percentage of NK cells (CD3- CD56+, top left panel), T regs (CD4+ CD25+FoxP3+, top right panel), CD4+ T cells (CD4+, bottom left panel) and CD8+ T cells (CD8+, bottom right panel) from live cells in AC or SCC tumor (Tu) and non-tumor healthy lung tissue (NTu) was estimated by flow cytometry.
   (C) The percentage of CD4⁺ T cells from CD3+ cells (CD4+, top left panel), of CD4⁺ T cells from CD3+ cells (CD8+, top right panel), memory CD4⁺ T cells (CD45RO+ CD4+, bottom left panel) and memory CD8+ T cells (CD45RO+CD8+, bottom right panel) from live cells in AC or SCC tumor (Tu) and non-tumor healthy lung tissue (NTu) was estimated by flow cytometry.
Figure 3: Intratumoral INF-y producing CD4⁺ and CD8⁺ T cells were significantly inhibited in SSC tumors compared to non-tumoral tissue and tumors in AC patients.
   The percentage of IFN-γ⁺ cells from CD4⁺ T cells (top panel) and IFN-γ⁺ cells from CD8⁺ T cells from live cells in AC or SCC tumor (Tu) and non-tumor healthy lung tissue (NTu) was measured by flow cytometry.
Figure 4: Cytokine production in tumor and non-tumoral tissue of NSCLC patients.
   After cell stimulation with PMA+ ionomycin for 24h, the production of GM-CSF, IL-1β, IL-2 (A) and TNF-α, IL-23, IL-10 (B) in AC or SCC tumor (Tu) and non-tumor healthy lung tissue (NTu) were determined by Luminex.
Figure 5: Presence of M-MDSC and Tregs in tumors, and their influence on the tumor microenvironment. Inhibition of CD3ζ expression in T cells and mRNA for ARG1 in PBMC.
   (A) The percentage of M-MDSC (CD15⁻ CD14⁺ CD33^{hi} HLA-DR^{-/lo}, left panel) and Tregs (right panel) among live PBMC from healthy aged-matched controls patients, AC patients and SCC patients was measured by flow cytometry.
   (B) The amount of CD3ζ⁺ CD4⁺ T cells (left panel) and CD3ζ⁺ CD8⁺ T cells (right panel) among live PBMC from healthy aged-matched controls patients, AC patients and SCC patients was measured by flow cytometry.
   (C) The relative expression of ARG1 was measured by qPCR from mRNA extracted from PBMC from healthy control, AC patients and SCC patients
Figure 6: Correlation analysis between the different types of immune suppressive cells in AC and SCC.
   Correlation analysis between the number of M-MDSC (CD15⁻ CD14⁺ CD33^{hi} HLA-DR^{-/lo}) and Tregs number in PBMC from AC patients (top left), SCC patients (top right) and healthy aged-matched controls patients (bottom left).

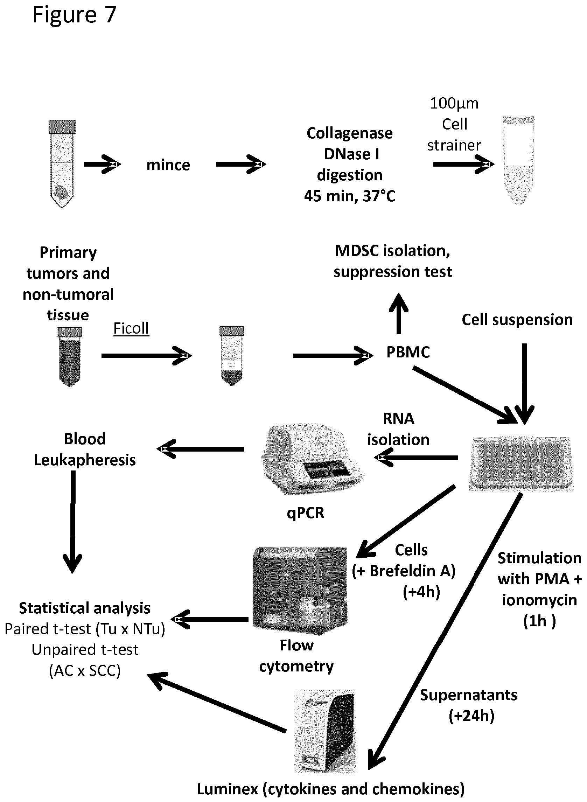

Figure 7: Experimental protocol

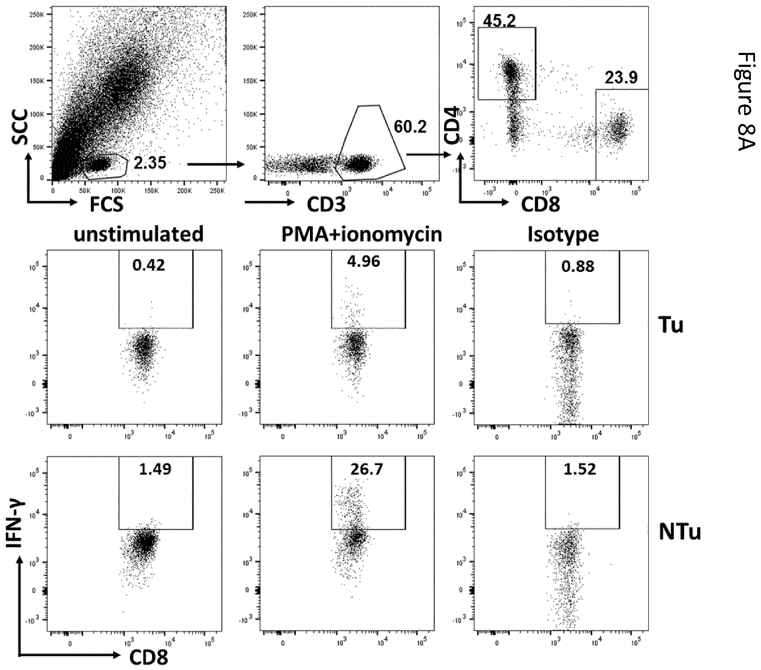

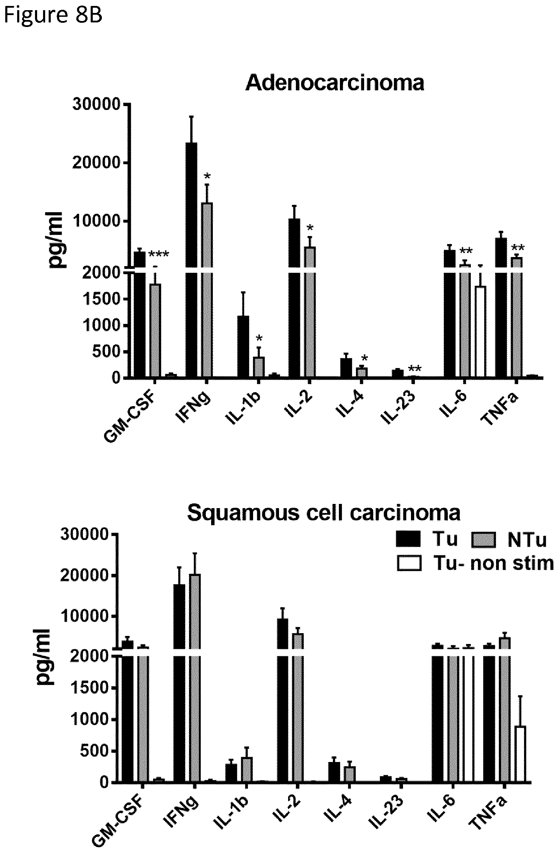

Figure 8: Intra-tumoral T cells and NK cells are functionally more suppressed in SCC than in AC NSCLC patients.
   Tumor and non-tumoral cell suspensions were stimulated with PMA and ionomycin for 1h before Brefelden was added for additional 3h. Then cells were stained for intracellular IFN-γ and analyzed by flow cytometry;
   (A) Gating strategy to detect IFN-γ-producing CD8⁺ and CD4⁺ T cells.
   (B) Cytokine production determined after 24h of stimulation with PMA+ionomycin by luminex. Graphs represent means of n= 43 AC and n= 39 SCC (* p < 0.05; ** p < 0.01, *** p < 0.005 or **** p < 0.005).

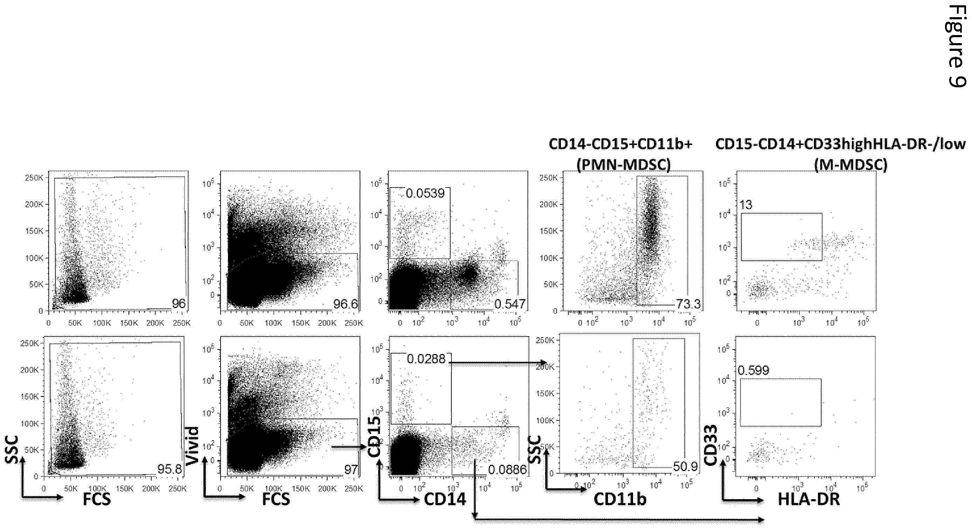

Figure 9: Gating strategy to detect intro-tumoral Tregs, polymorphonuclear- MDSC (PNM-MDSC) (CD14⁻CD15⁺CD11b⁺) and monocytic-MDSC (M-MDSC) (CD15⁻CD14⁺CD33^{hi} HLA-DR^{-/low}).

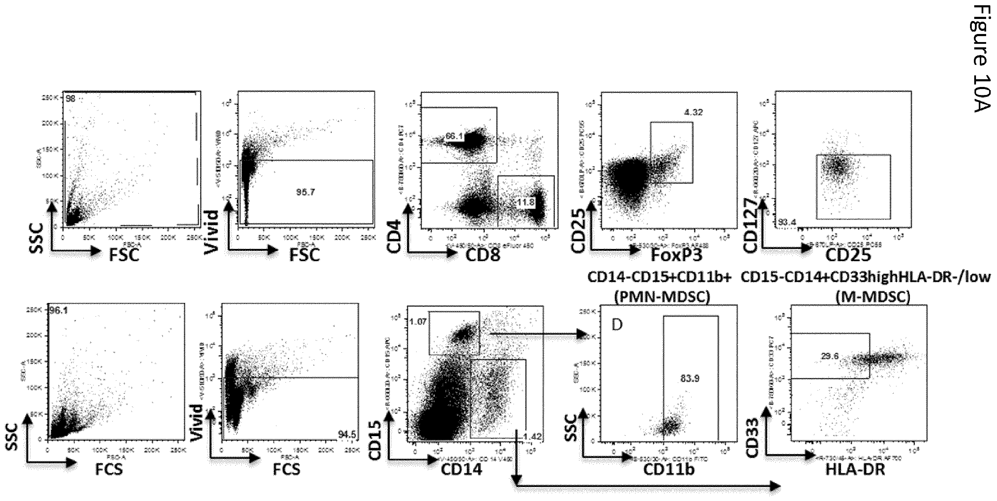

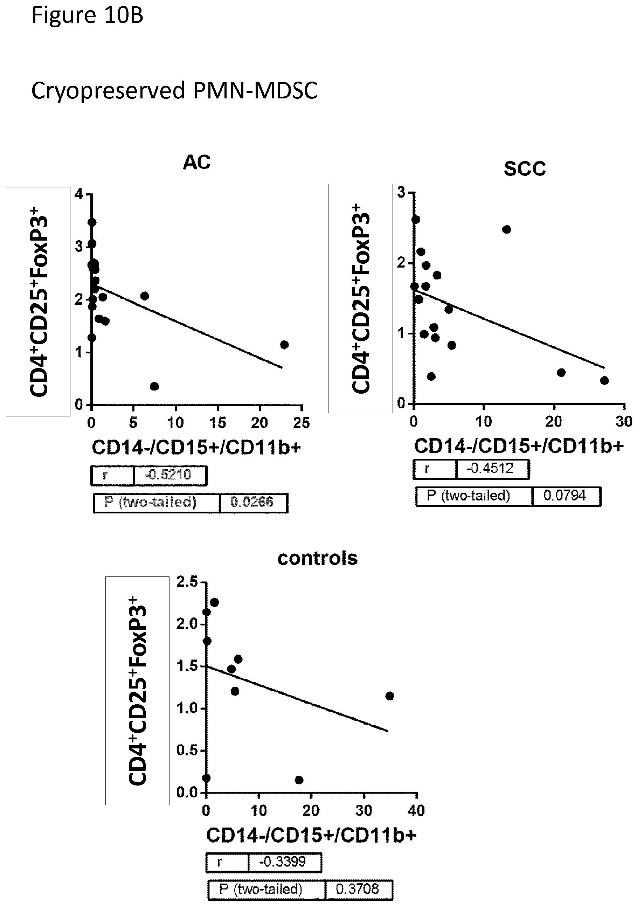

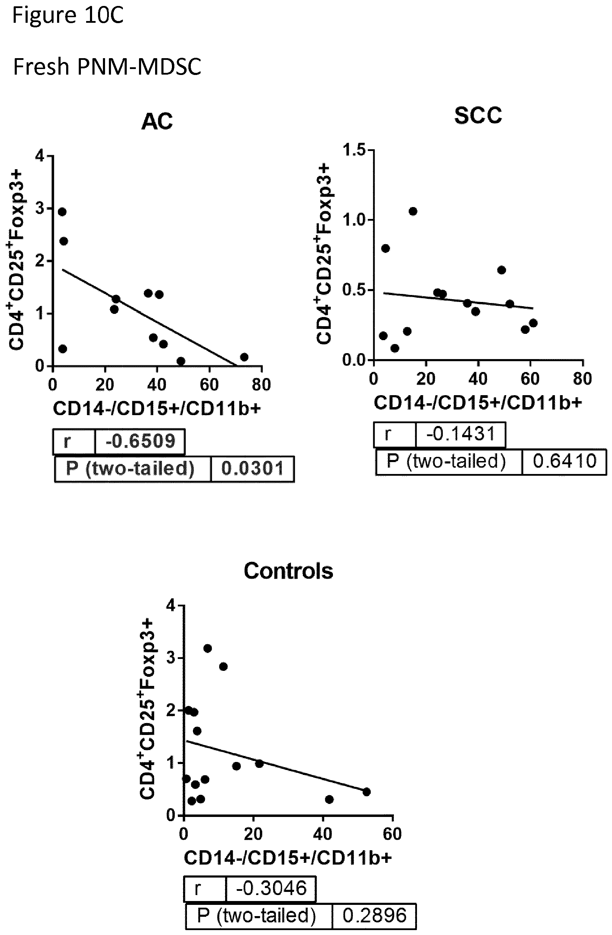

Figure 10: Tregs are more abundant in blood of AC patients whereas the number of MDSC is higher in blood of SCC patients
   A) Gating strategy to detect Tregs, PNM-MDSC (CD14⁻CD15⁺CD11b⁺) and M-MDSC (CD15⁻CD14⁺CD33^{hi}HLA-DR^{-/low} in the blood of NSCLC patients. Quantitative analysis of MDSC in PBMCs from cryopreserved PBMC
   (B-C) Correlation between the amount of Tregs and PMN-MDSC in PBMC of NSCLC patients from cryopreserved (B) and fresh (C) samples.

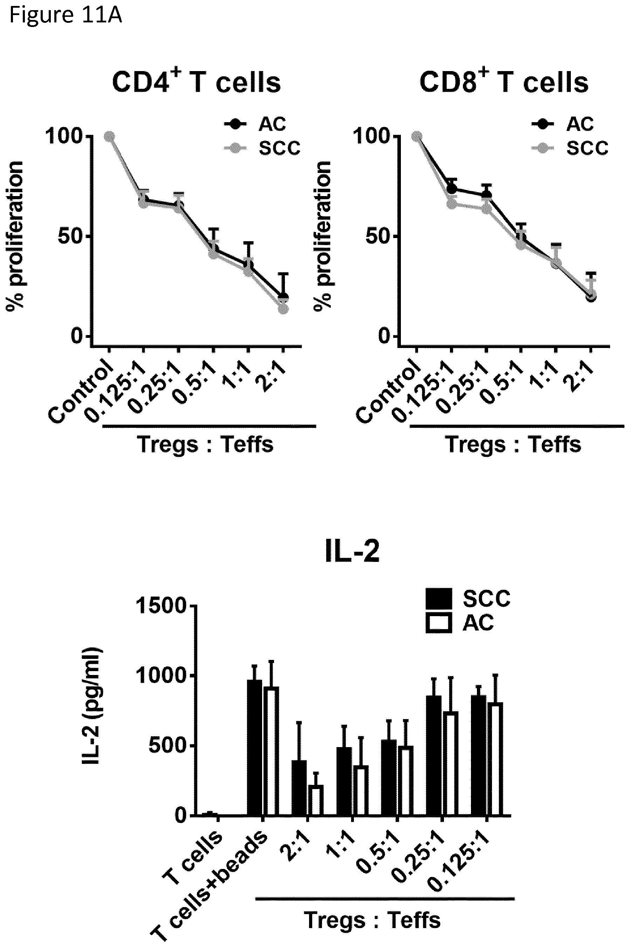

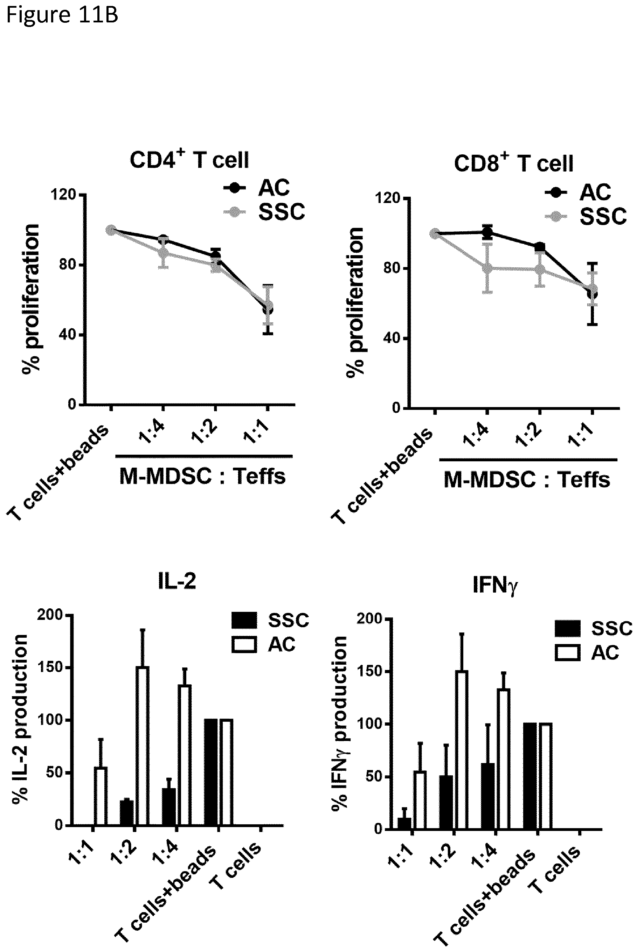

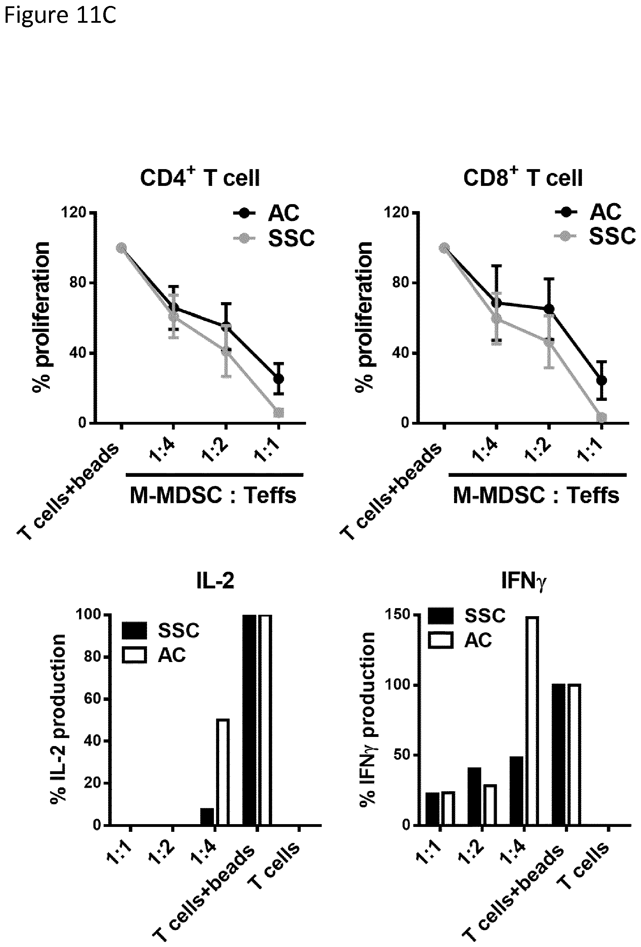

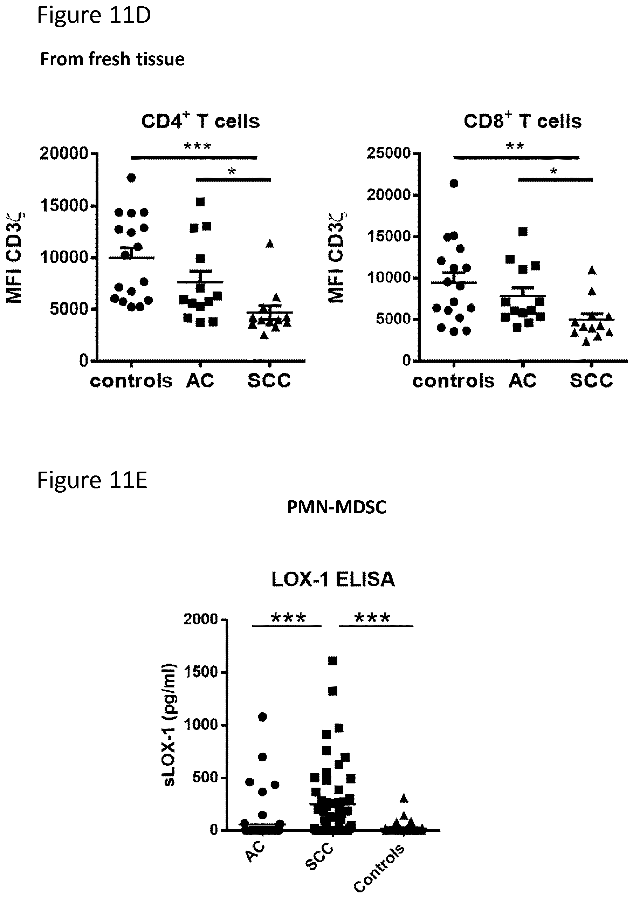

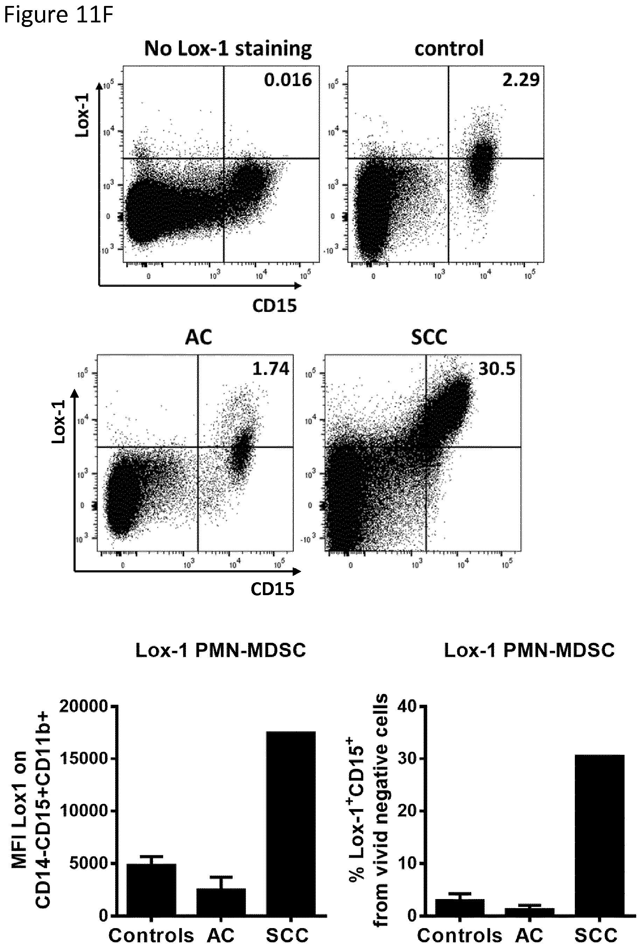

Figure 11: Suppressive function of Tregs, but not MDSC is comparable in blood of AC and SCC NSCLC patients
   (A) Treg suppression assay .
   (B) M-MDSC suppression assay - magnetic beads isolation, or
   (C) cell sorting , including IL-2 and IFN-γ secretion detected by ELISA.
   (D) Flow cytometric detection of CD3ζ expression in T cells from fresh samples of NSCLC patients.
   (E) LOX-1 protein was detected in plasma samples by ELISA. Graphs represent means n= 56 AC, n= 52 SCC and n=41 control (* p < 0.05; ** p < 0.01,*** p < 0.005 or **** p < 0.005).
   (F) LOX-1 positive PMN-MDSCs were quantified by flow cytometry. Graphs represent means n=3 AC, n=1 SCC and n=2 control.

### Examples

**Table 1: Tumor Samples**

| | Blood | Tumor Lung tissue Blood | Total |
|---|---|---|---|
| Total samples | 49 | 94 | 143 |
| Adenocarcinoma (AC) | 13 | 38 | 51 |
| Squamous cell carcinoma (SCC) | 12 | 35 | 47 |
| Large cell carcinoma (LC) | 1 | 1 | |
| Other | 13 | 16 | |
| Benign | 10 | 4 | |

### Example 1

Cells isolated from NSCLC tumors displayed high viability, however cells isolated from tumors were significantly less viable in comparison to cells isolated from non-tumoral tissue (see Figure 1A top panel). Squamous cell carcinoma (SCC) contained higher level of epithelial cells (determined by FITC labeled human epithelial antigen and pan-cytokeratin) compared to adenocarcinoma (AC) (see Figure 1A bottom panel). The infiltration of CD45⁺ lymphoid cells was significantly higher in tumor than in non-tumoral tissue (see Figure 1B). Cells were determined as % of DAPI negative total cell count (see Figure 1C gating strategy).

### Example 2

The infiltration of all immune cell populations tested was comparable between AC and SCC histological subtypes (see Figure 2). The immune cell infiltration was higher in tumoral than non-tumoral tissue. When not stated the percentage of immune cell population was determined from viable (DAPI negative) total cell count.

### Example 3

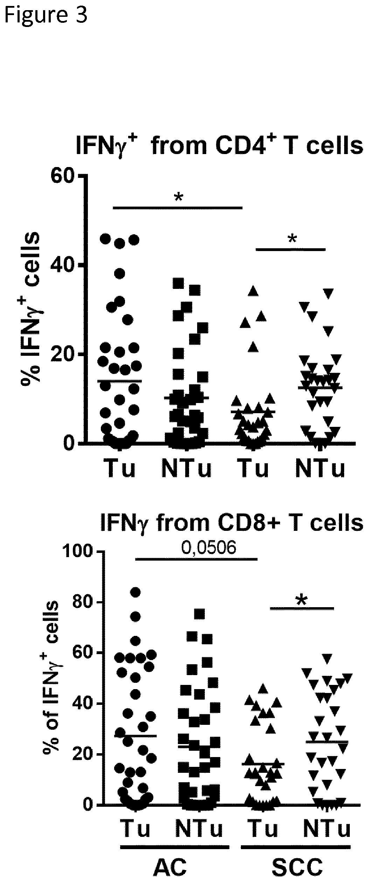

Intratumoral INF-γ producing CD4+ and CD8+ T cells (see Figure 3 top and bottom panel, respectively) were significantly inhibited in SSC tumors compared to non-tumoral tissue and tumors in AC patients.

### Example 4

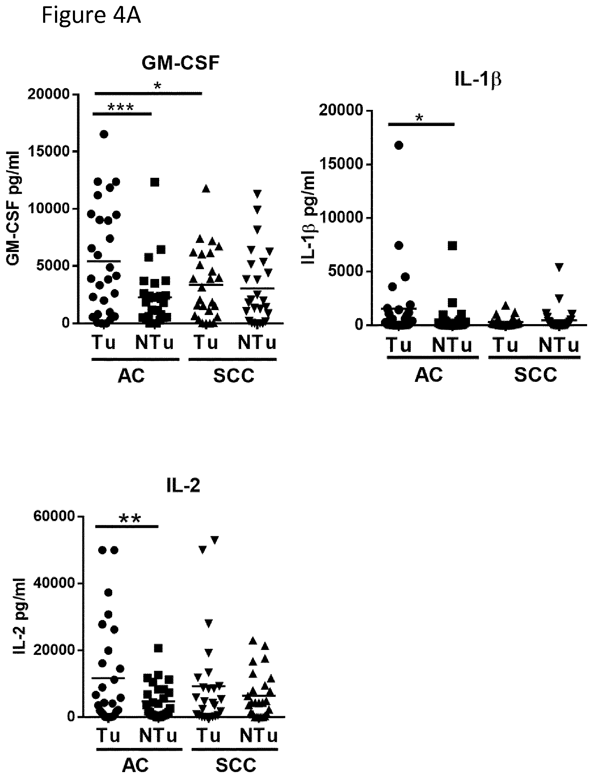

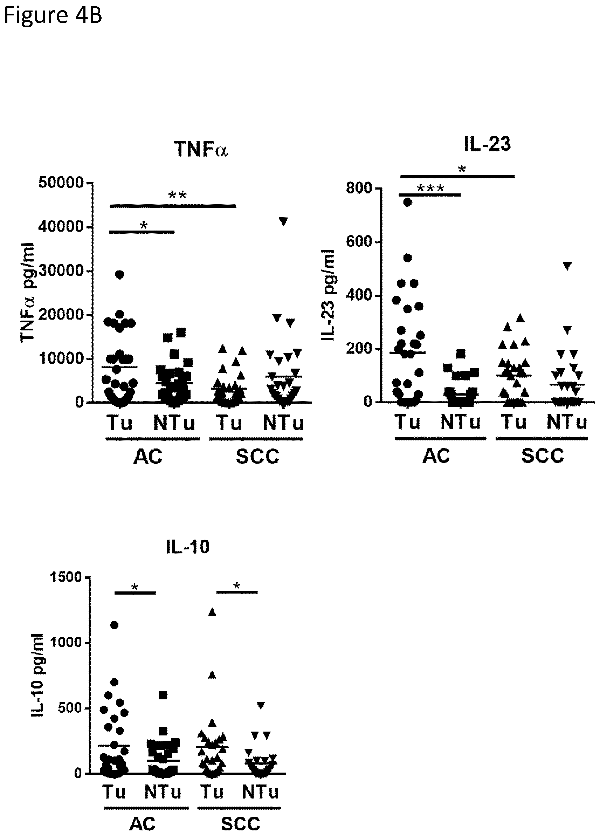

Cell suspensions were stimulated with PMA+ ionomycin for 24h and cytokines were determined by Luminex. There was a significantly higher production of pro-inflammatory cytokines such as GM-CSF, IL-1β, IL-2 and TNF-α in tumors from AC patients over respective non-tumoral tissues (see Figure 4A and B). Similarly, GM-CSF and TNF-α production is higher in tumors from AC patients than in tumors from SCC patients. This was observed also for IL-23 (see Figure 4A and B). IL-10 was significantly enhanced in tumors from both NSCLC subtypes (see Figure 4B). No tumor-associated production of IFN-γ, IL-12p70, IL-13, IL-17, IL-22, IL-9, IL-21, IL-4, IL-6 was observed compared to non-tumoral tissue.

### Example 5

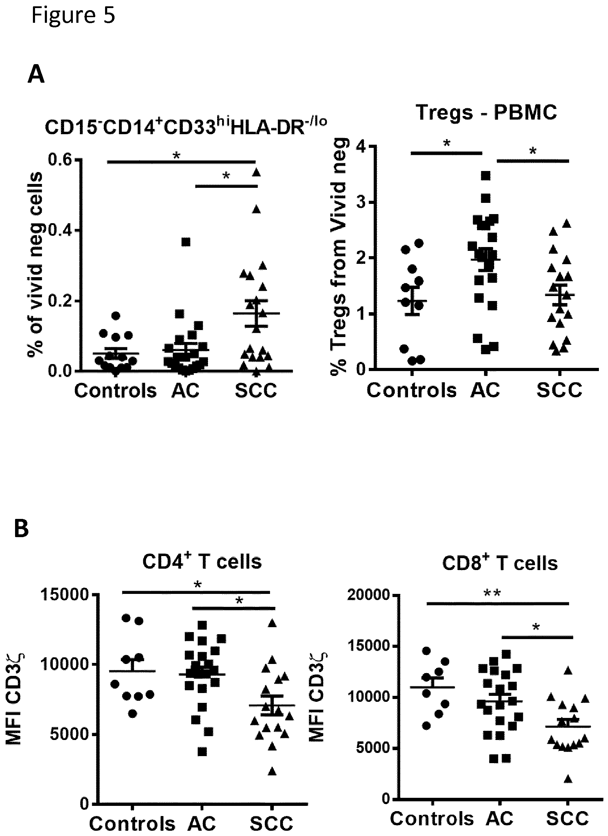

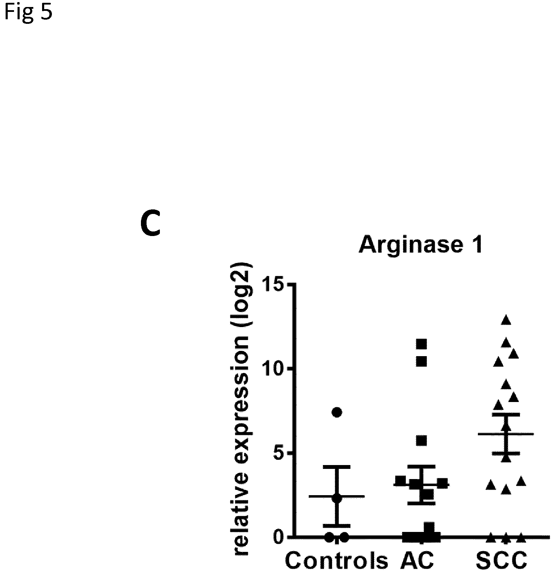

M-MDSC (CD15⁻CD14⁺CD33^{hi}HLA-DR^{-/lo}) are abundant in blood of SCC patients whereas T regulatory cells (CD4⁺CD25⁺Foxp3⁺ CD127^{low}) are elevated in AC patients (see Figure 5A). Down-regulation of CD3ζ chain in T cells (see Figure 5B) and elevated levels of ARG1 are induced in SCC but not in AC patients (see Figure 5C).

### Example 6

The percentage of the M-MDSC (CD15⁻CD14⁺CD33^{hi}HLA⁻DR^{-/lo}) population negatively correlates with the percentage of Tregs (CD4⁺CD25⁺Foxp3⁺CD127^{low}) in the blood of AC patients but not of SCC patients or age-matched healthy controls (see Figure 6). These data suggest that CD4⁺CD25⁺Foxp3⁺CD127^{low} Tregs might represent the major immunosuppressive population in NSCLC patients with AC.

### Example 7: Materials and Methods

**Table 2: Overview of NSCLC patients**

| | | Adenocarcinoma (AC) | Squamous cell carcinoma (SCC) | Age-matched donors (controls) |
|---|---|---|---|---|
| Sex (male/female) | | 16/27 | 34/5 | 10/7 |
| Age, year (mean±SD) | | 63 ± 11 | 67 ± 9 | 62 ± 10 |
| Tumor tissue + non-tumoral tissue | | 43 | 39 | - |
| TMN Stage | | | | - |
| | IA | 9 | 6 | |
| | IB | 10 | 9 | |
| | IIA | 5 | 8 | |
| | IIB | 4 | 6 | |
| | IIIA | 12 | 8 | |
| | IIIB | 1 | 0 | |
| | IV | 2 | 0 | |
| Blood | | 32 | 30 | 17 |
| Plasma | | 30 | 30 | 30 |

The experimental protocol is outlined in Figure 7.

### Processing of primary tumors and non-tumoral tissue from NSCLC patients

Tumoral (Tu) and non-tumoral tissue (NTu) were obtained from 82 NSCLC patients undergoing neoadjuvant surgery. The characteristics of NSCLC patients are depicted in Table 2. Tissue samples obtained at the day of surgery were chopped into small pieces and incubated with agitation in RMPI 1640 medium (Gibco) with 100 ng/ml DNAse I and 20 ng/ml Collagenase D (both from Roche) for 45 minutes at 37°C. The cell suspension was then passed through the 100 µm strainer into 50 ml falcon tubes to obtain single cell suspensions. The infiltrated immune cell populations were analyzed by flow cytometry immediately after staining with specific antibodies for 30 min at 4°C as described below. For further stimulation lymphocytes were counted and seeded into 96-well plates at the concentration of 1×10⁶ lymphocytes/ml in RPMI-1640 medium supplemented with 10% heat-inactivated fetal bovine serum (PAA), 2 mM GlutaMAX I CTS (Gibco) and 100 U/ml penicillin + 100 mg/ml streptomycin (Gibco). Cells were stimulated with 50 ng/ml PMA and 10 ng/ml ionomycin (both from Sigma-Aldrich) for 1h at 37°C before Brefeldin A (BioLegend, 1000x) was added for 3h to detect intracellular IFN-γ in CD8⁺ and CD4⁺T cells. In some experiments recombinant IL-15 (Peprotech, 13 ng/ml) was added to the cell culture and the proliferating CD8⁺ T cells and NK cells were detected by flow cytometry after 3 and 7 days of incubation at 37°C. Cell viability was detected by DAPI (Thermo Fisher Scientific) or by LIVE/DEAD^{®} Fixable Aqua Dead Cell Stain Kit 405 nm excitation (Invitrogen).

### PBMC isolation and plasma collection

2-3 tubes of peripheral blood collected in VACUETTE^{®} 9 ml K3 EDTA were obtained from 60 NSCLC patients undergoing neoadjuvant surgery and from 17-30 age-matched volunteers with no history of a malignant disease. 2 - 4 ml of plasma was collected after centrifugation of the peripheral blood at 3000 rpm for 5 minutes and stored at -80°C. Peripheral blood mononuclear cells (PBMC) were isolated by Ficoll-Pague gradient centrifugation. PBMCs were counted and seeded into the 96-well plate at the concentration of 1×10⁶ PBMCs/ml in complete medium or lysed in RLT buffer for mRNA preservation as described above. Freshly isolated PBMC were analyzed for Tregs and MDSC content by flow cytometry immediately after staining with specific antibodies for 30 min at 4°C as described below. Some PBMCs were cryopreserved in CryoStor^{®} CS10 (BioLife Solution) in liquid nitrogen before analyses.

### Antibodies used for immune cell analyses and staining protocol

Epithelial tumor cells - CD45 PE-DyLight594 (Exbio), anti-pan cytokeratin AlexaFluor 488 (eBioscience), anti-human epithelial antigen-FITC (DAKO).

Dendritic cells: Lin neg (CD3-FITC, CD19-FITC, CD20-FITC, CD56-FITC), CD45-PE-DyLight594, CD11c-APC (all from Exbio), HLA-DR-PE-Cy7 (BD Pharmingen^{™}).

Lymphocytes/NK cells: CD3-AlexaFluor 700, CD8-PE-Cy7, CD19-FITC, CD20-FITC (all from Exbio), CD4-ECD (Beckman Coulter), CD56-PerCP/Cy5.5 (eBioscience).

Naive/memory T cells: CD3-AlexaFluor 700, CD8-PE-Cy7, CD45RA-PE, CD45RO-APC, CD62L-FITC (all from Exbio), CCR7-PerCP/Cy5.5 (BioLegend), CD4-ECD (Beckman Coulter).

IFN-γ producing T cells: CD3-Alexa 700, CD8-PE-Cy7 (both from Exbio), CD4-ECD (Beckman Coulter), IFN-γ-FITC (BD Pharmingen).

T regulatory cells in tumors: CD8-PE-Cy7 (Exbio), CD4-ECD (Beckman Coulter), Foxp3-AlexaFluor 488 (eBioscience).

T regulatory cells in PBMC: CD4-PE-Cy7, CD8-eFluor 450, CD25-PerCP-Cy5.5, CD127-APC, Foxp3-AlexaFluor 488, CD3ζ-PE (all from eBioscience), Ki-67-AlexaFluor 700 (BD Pharmingen).

MDSC: CD14-BD Horizon V450 (BD Horizon), HLA-DR-Alexa Fluor 700, CD33-PE-Cy7, (BioLegend), CD11b-FITC, CD15-APC (eBioscience) + possibly LOX1- PE (BioLegend).

Cells were stained extracellularly with the mixture of appropriate antibody in PBS for 30 min at 4°C. For intracellular staining of IFN-γ, Foxp3, CD3ζ and Ki-67 the cells were fixed for 30 min using Fixation Buffer (eBioscience), permeabilized with Permeabilization Buffer (eBioscience) and stained intracellularly for 30 min at 4°C. Cells were washed with PBS and analyzed by LSRFortessa (BD Biosciences). Data were analyzed with FlowJo software (Tree Star). Flow cytometry data may be expressed as mean fluorescent intensity (MFI).

### Cytokine production and LOX-1 plasma detection

To determine cytokine production, cell supernatants were harvested 24 h after stimulation with PMA and ionomycin or harvested at day 3 and 7 after stimulation with IL-15 as described above. Cell culture supernatant was stored at -80°C. GM-CSF, IFN-γ, IL-10, IL-12p70, IL-13, IL-17, IL-22, IL-9, IL-1β, IL-2, IL-21, IL-4, IL-23, IL-6, TNFα were determined using Luminex assay (MILLIPLEX^{™} MAP Human Th17 Magnetic Bead Panel, Merck Millipore) by MagPix (XMAP Technology, Luminex). LOX-1 protein as a marker of PMN-MDSC presence was detected in plasma samples (diluted 1:10 or 1:50) from NSCLC patients by ELISA (RD System).

### Isolation of CD33⁺HLADR⁻ MDSC with magnetic microbeads

CD33⁺HLADR⁻ cells were isolated from the PBMC obtained from leukapheresis of NSCLC patients using MACS microbeads and columns (Miltenyi Biotec). Briefly, thawed PBMC were resuspended in cold MACS buffer and incubated with HLA-DR microbeads (Miltenyi Biotec) for 15 min on ice. Then cells were washed with cold MACS buffer to remove unbound beads and subsequently subjected to depletion of HLA-DR⁺ cells on MACS column according to manufacturer's instructions. The negative cell fraction was collected, washed and then incubated with CD33 microbeads. MACS column was used for positive selection of CD33⁺HLA-DR⁻ cells. The purity of the CD33⁺ cell population was evaluated by flow cytometry and exceeded 90%.

### Isolation of CD33⁺CD14⁺HLA-DR⁻ MDSC by cell sorting

Briefly, PBMC were isolated from leukapheresis of NSCLC patients by using Ficoll Paque and stored at -80°C. Thawed PBMC were resuspended in cold MACS buffer and incubated with CD33 microbeads (Miltenyi Biotec) for 15 min on ice. Then cells were washed with cold MACS buffer to remove unbound beads and subsequently subjected to depletion of CD33 negative cells on MACS column according to manufacturer's instructions. The CD33⁺ cell fraction was collected, washed and stained with anti-CD14 and anti-HLA-DR Ab for 20 min at 4°C. Cells were than washed with PBS and the CD33⁺ CD14⁺ HLADR^{-/low} MDSCs were subsequently sorted using S3e cell sorter (Biorad).

### MDSC suppression assay

Purified autologous T cells (50 000 cells per well) were labeled with CFSE, activated using anti-CD3/CD28 expander beads (2.5 × 10⁵ beads per well) and incubated in the presence of different ratios of magnetic beads-purified or sorted MDSC (1:1, 1:2, 1:4 T cell/MDSC ratio). T-cell proliferation was measured as CFSE dilution using flow cytometry on day 6. Suppression is calculated as % of controls = (proliferation of analyzed sample - proliferation of non-proliferating cell) / (proliferation of control - proliferation of non-proliferating cells). The production of IFN-γ and IL-2 in cell culture supernatants was evaluated by ELISA (RD System).

### Isolation of T regulatory cells and suppression assay

Thawed PBMC from NSCLC leukapheresis were resuspended in cold PBS and passed through 30 µm strainer to remove cell clumps before isolation. CD25⁺CD4⁺CD127^{low} Tregs were isolated using EasySep Human CD4⁺CD127^{low}CD25⁺regulatory T cell isolation kit (Stemcell Technologies). CD3⁺ effector cells were isolated by using EasySep Human T cell enrichment kit (Stemcell Technologies) and stained with CFSE (1 µM, Invitrogen). The purity and CFSE-staining was confirmed by flox cytometry. For suppression assay CFSE-stained CD3⁺ T effector cells (50 000 cells per well) were seeded in 96-well plate alone (negative control), activated using anti-CD3/CD28 expander beads (16.7 × 10³ Dynabeads per well - ratio 3:1 T cells/beads) only (positive control) or activated with beads and incubated in the presence of different ratios of purified Tregs (2:1, 1:1, 0.25:1, 0.125:1 Tregs/Teff ratio). Cells were incubated in complete RPMI 1640 supplemented with 10% human AB serum (200 µl/well). T cell proliferation was analyzed on day 3 (optimal proliferation of controls - 60-80%, at least 3 generations). Suppression is calculated as described above for MDSC suppression assay. The production of IL-2 in cell culture supernatants was evaluated by ELISA (RD System).

### qPCR and proteomics analysis

Total RNA was isolated using an RNeasy Mini Kit (Qiagen). Each sample containing RLT buffer and cell lysate was quickly thawed and processed in accordance with the manufacturer's protocol which included a DNase I digestion step. The RNA concentration and purity were determined using a NanoDrop 2000c (Thermo Scientific), and the RNA integrity was assessed using an Agilent 2000 Bioanalyzer (Agilent). Purified RNA samples were stored at -80°C until further use. cDNA was synthesized from 100 ng of total RNA using an iScript cDNA Synthesis Kit (BioRad). Expression of ARG1 gene was determined by qPCR on CFX96 Touch^{™} Real-Time PCR Detection System (BioRad). Each 10 µl reaction contained 5 µl of KAPA PROBE FAST qPCR Master Mix (Kapa Biosystems), 0.5 µl of each forward and reverse primers (500 nM each; TIB Molbiol,), 0.5 µl of TaqMan probe (200 nM; TIB Molbiol), 1.5 µl of RNase-free water and 2 µl of 5x diluted cDNA. Each reaction was done in triplicate. The temperature cycling protocol was following: 3 minutes at 95°C followed by 45 cycles (95°C for 15 s and 60°C for 60 s). The formation of PCR products of the expected lengths was confirmed by agarose gel electrophoresis. The Cq values were determined using CFX Manager software (BioRad) and the relative expressions of the studied genes were calculated with GenEx software (MultiD Analyses) with cut off at 36 cycle. Proteomics analyses were conducted from genes published in NSCLC cancer tumor samples in Cancer Genome Atlas. Immune cell population were analyzed using transcriptome-based computational microenvironment cell populations-counter (MCP-counter) method introduced by Becht et al. (2016) from n= 508 AC and n= 495 SCC patient' tumor samples.

### Statistical analysis

Two-tailed paired t-test or unpaired, non-parametric Mann-Whitney test were applied for data analysis using GraphPad PRISM 6 (San Diego, California, USA). The results were considered statistically significant if * p < 0.05, ** p < 0.01 or *** p < 0.001. Data were expressed as mean ± SEM.

### Example 8: T cells, B cells and NK cell infiltration is similar in AC and SCC tumors, but myeloid DC (CD11c⁺HLA-DR^{hi}) are significantly decreased in SCC tumors

Immune cell infiltration is higher in NSCLC tumors than in adjacent non-tumoral tissue. Possible differences in T cell, B cell and NK cell or dendritic cell infiltration between two histologically distinct tumors in adenocarcinoma (AC) and squamous cell carcinoma NSCLC patients (SCC) were analyzed. Single cell suspensions from tumors and non-tumoral tissue obtained from 42 AC and 39 SCC neoadjuvant NSCLC patients (Table 2) were analyzed by flow cytometry. Cell viability was higher in non-tumoral than in tumoral tissue, but tumor cell viability was on average around 85% (see Figure 1A top panel). Higher infiltration of CD8⁺ and CD4⁺ T cells with predominantly memory phenotype, B cells, NK cells and DC in tumors was observed when compared to non-tumoral tissue (see Table 3). Infiltration rates of these immune cell populations between AC and SCC tumors were not statistically different..

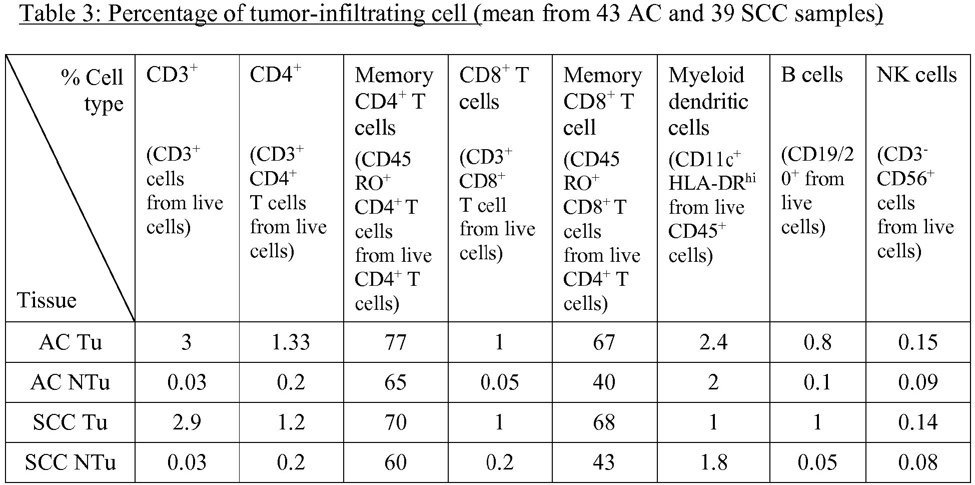

### Example 9: Intra-tumoral T cells and NK cells are functionally more suppressed in SCC than in AC NSCLC patients

Since phenotypic analysis of tumor-infiltrated immune cells offers only quantitative evaluation of immune cell infiltration, tumor and non-tumoral cell suspensions were stimulated with PMA and ionomycin for 1h, followed by addition of Brefeldin A for additional 3h. IFN-γ production in CD8⁺ and CD4⁺ T cells was subsequently determined by flow cytometry. The gating strategy is shown in Figure 8A. The capacity of tumoral and non-tumoral CD8⁺ and CD4⁺ T cells to produce IFN-γ after non-specific stimulation was similar in T cells form AC and SCC patients (see Table 4). However, the IFN-γ production capacity of SCC tumors CD8⁺ and CD4⁺ T cells was impaired when compared to T cells coming from adjacent healthy tissue, suggesting that T cells in SCC tumors might be more suppressed in their IFN-γ-mediated effector functions than T cells in AC tumors (see Table 4). Default IFN-γ production was not observed in T cells in non-stimulated tissues.

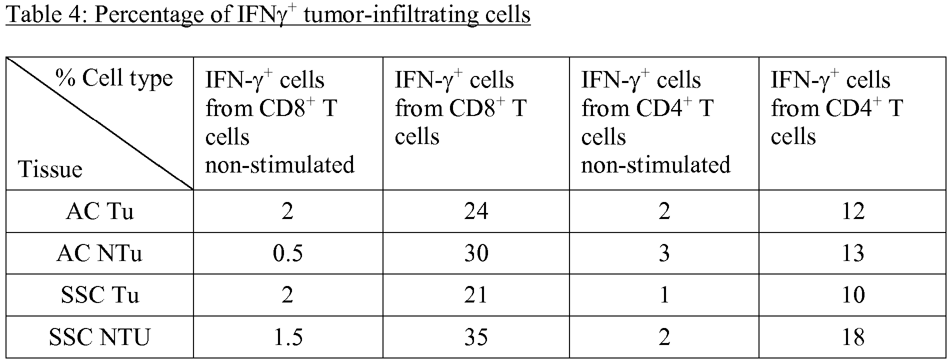

Furthermore, the cytokine production from tissues stimulated for 24 h (see Figure 8B) was analyzed by Luminex. The production of GM-CSF, IFN-γ, IL-1β, IL-2, IL-4, IL-23, IL-6 and TNF-α was significantly higher in AC tumors when compared to adjacent non-tumoral tissue. This might be due to a higher lymphocyte (see Figure 1B) and epithelial cell (see Figure 1A bottom panel and Table 5) content in AC tumor tissue than in non-tumoral tissue. However, in SCC tumors the cytokine production, the majority of them being pro-inflammatory immune cytokines, was comparable with non-tumoral tissue, suggesting a higher suppression of pro-inflammatory cytokines after stimulation in SCC tumors when compared to AC as the leucocytes infiltration and the number of epithelial cells in tumors was comparable. There was no cytokine production observed in non-stimulated tissues (see Figure 8B).

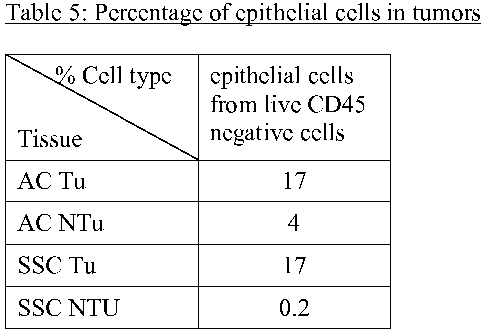

As PMA and ionomycin represent strong unspecific stimulation of immune cells, IL-15 was used to activate predominantly NK cells and CD8⁺ T cells, playing a major role in antitumor immunity. Tumor cell suspensions were incubated alone or with IL-15 for 7 days. The proliferation of CD8⁺ T cells and NK cells was analyzed on Day 0, 3 and 7 by Ki67⁺ staining followed by flow cytometry. Whereas 68% and 79% of T cells and NK cells, respectively, proliferated on Day 3 of incubation in AC tumors, only 23% and 8% of T cells and NK cells, respectively, proliferated in SCC tumors, confirming a higher immunosuppressive environment in SCC than AC tumors (see Table 6).

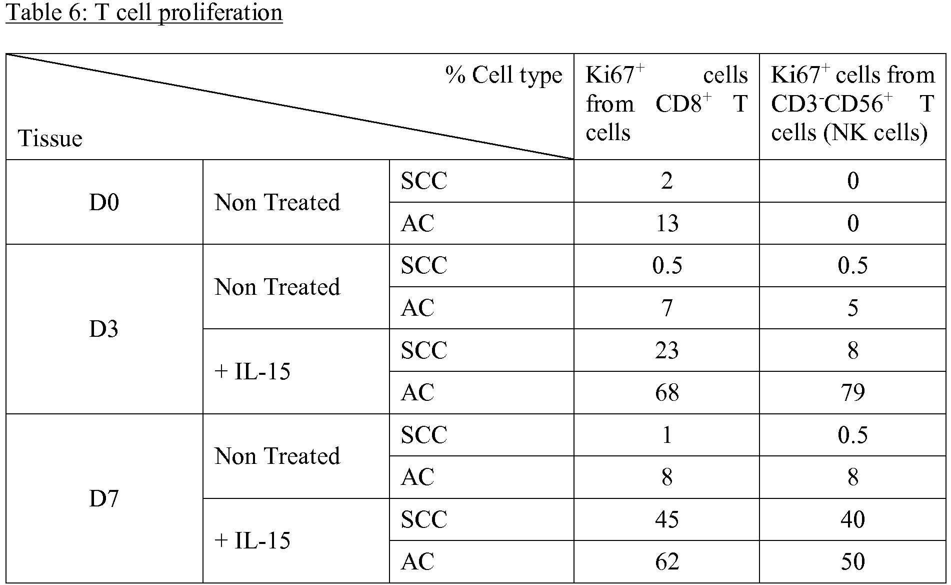

Immune genes expression analyzed from the TCGA database (The Cancer Genome Atlas, National Cancer Institute and National Human Genome Research Institute, https://cancergenome.nih.gov, see Table 7) show higher expression of antitumor-related immune genes in AC than in SCC which again supports higher immunosuppressive microenvironment in SCC over AC. Interestingly, more antigens is expressed in SCC than AC.

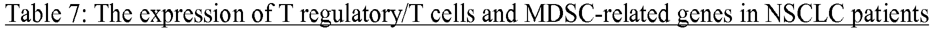

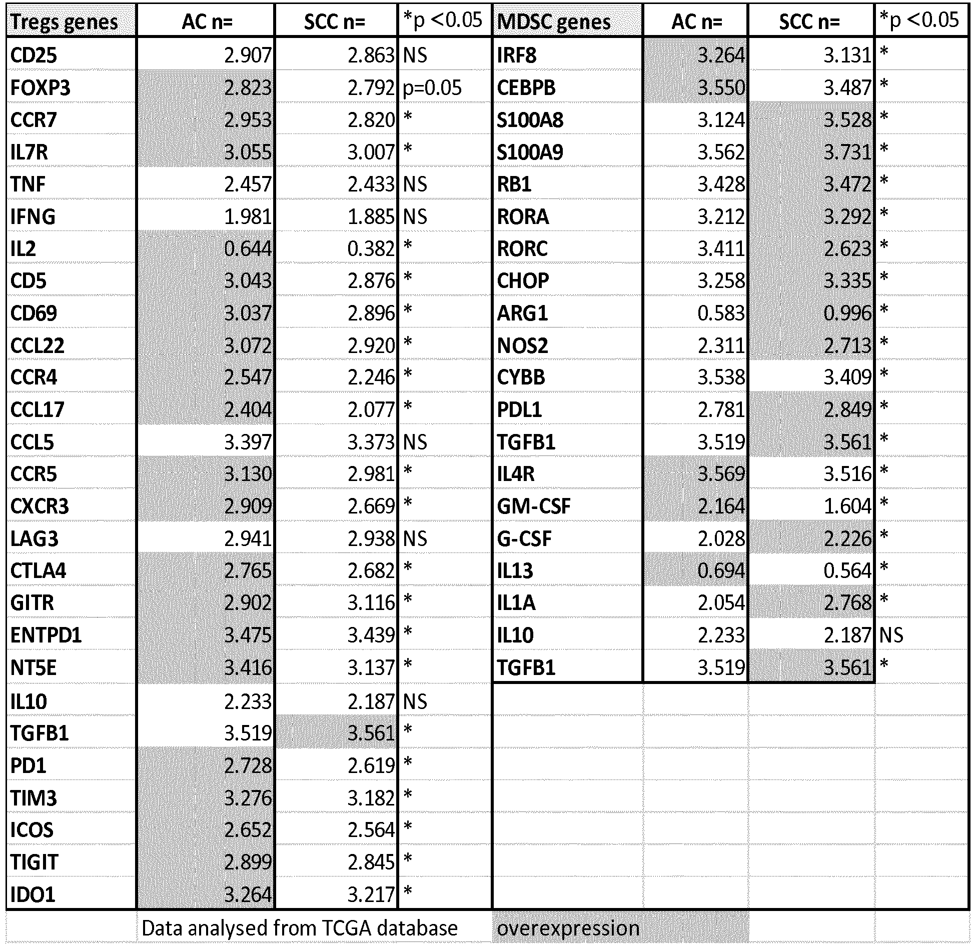

### Example 10: T regulatory cells infiltration is greater in AC tumors whereas SSC tumors are infiltrated more by myeloid-derived suppressor cells

In view of the differences between the immunosuppressive tumor microenvironment of AC and SCC patients two major immunosuppressive immune cell populations - CD4⁺CD25⁺ Foxp3⁺CD127^{low} Tregs and MDSC, here specifically PMN-MDSC and M-MDSC were analyzed. Fresh tissue samples were analyzed by flow cytometry. The gating strategy is shown in 9 and was, together with antibody staining for MDSC, adopted from Walter et al. (2012) and Bronte et al. (2016). Both Tregs and MDSC populations were detected in higher numbers in tumors than in non-tumoral tissue in both histological subtypes of NSCLC (see Table 8).

However infiltration of Tregs was higher in AC than SCC tumors (see Table 8). Conversely, a higher number of PMN-MDSC and M-MDSC was detected in SCC than in AC tumors (see Table 8). As MDSC cannot be defined solely by phenotypic markers and it is technically challenging to perform suppression assays from intratumoral MDSC, the expression of genes associated with MDSC characterization as described in Bronte et al. (2016) was analyzed (see Table 7). The expression of genes related to Tregs and/or T cell function from TCGA database on (n= 508 AC and n= 495 SCC patients) was also evaluated. Table 7 shows that most of the higher expression of most genes associated with MDSCs in SCC, a higher expression of genes associated preferentially with Tregs/T cells in AC tumors, confirming the phenotypic observation.

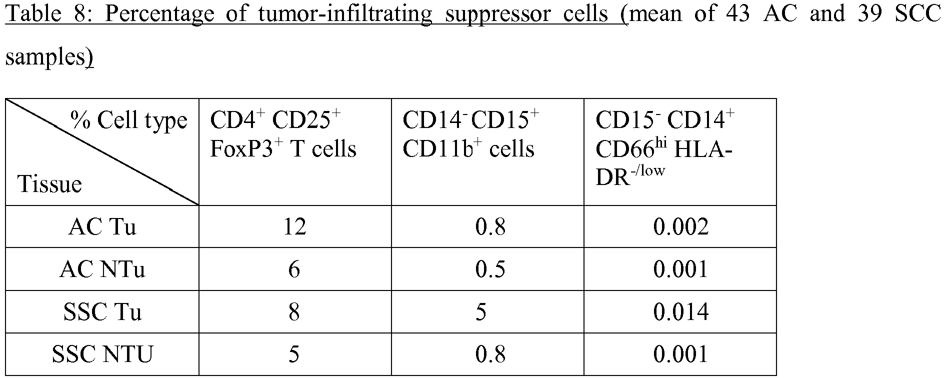

### Example 11: Tregs are more abundant in blood of AC patients whereas the number of MDSC is higher in blood of SCC patients

As Tregs infiltration was higher in AC and MDSC infiltration higher in SCC tumors, the presence of these cells was also analyzed in blood of NSCLC patients. The immunosuppressive population was quantitatively measured by flow cytometry in PBMCs isolated from NSCLC patients and age-matched donors with no history of malignant disease (see Table 2, Figure 5A). PBMCs wear also functionally tested by suppression assays (see Figure 11). The gating strategy is shown in Figure 10A. Tregs (CD4⁺CD25⁺Foxp3⁺CD127^{low}), PMN-MDSC (CD14⁻CD15⁺CD11b⁺) and M-MDSC (CD15⁻CD14⁺CD33^{hi}HLA-DR^{-/low}) were analyzed from frozen (see Figure 5A and Table 9) and from freshly isolated PBMCs (see Table 10). Tregs were more abundant in the blood of AC patients than in SCC patients and age-matched controls.

Moreover, Tregs detection was not affected by cryopreservation. Tregs suppression assays further showed that Tregs from both histological subtypes were similarly competent to decrease CD8⁺ and CD4⁺ T cell proliferation (see Figure 11A). This suggests only quantitative differences in Tregs in blood between AC and SCC patients. Interestingly, the number of Tregs correlated negatively with both MDSC populations only in blood of AC patients (see Figure 6 for M-MDSC and Figure 10BC for PMN-MDSC).

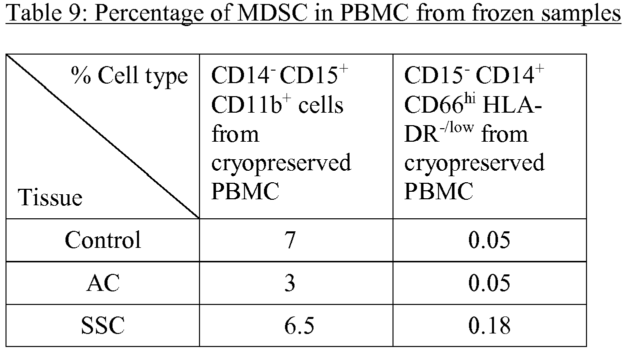

To the contrary to Tregs, the numbers of both types of MDSC are affected by cryopreservation. However, analysis of fresh PBMC samples showed more M-MDSC cells in the blood of SCC than AC patients and age-matched controls (see Table 10). The number of PMN-MDSC was comparable between AC and SCC patients and was higher than in age-matched controls. As PMN-MDSC cannot be distinguished from neutrophils with no suppressive function we analyzed LOX1 expression on these cells by flow cytometry (see Figure 11F). The presence of LOX-1 positive PMN-MDSC was largely increased in SCC patients, compared to AC patients and healthy controls. Furthermore, LOX-1 protein associated with the presence of PMN-MDSC was significantly increased in plasma from SCC patients in comparison to AC patients and healthy age-matched controls (see Figure 11E). Similarly, expression of ARG 1, an effector molecule predominantly expressed in PMN-MDSCs, was increased in SCC patients (Figure 5C). Immunosuppressive action of MDSCs was shown to inhibit CD3ζ expression in T cells. Indeed, CD3ζ chain expression was only inhibited in CD8⁺ and CD4⁺ T cells in SCC patients when compared to AC patients or age-matched controls (Figure 5B and Figure 11 D).

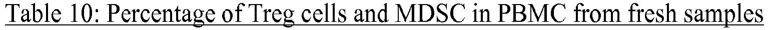

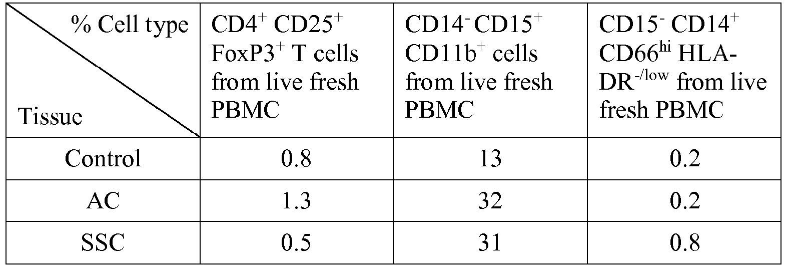

To prove that MDSCs found in SCC patients might be more suppressive than MDSCs found in AC patients MDSC suppression assays were performed (see Figure 11A-C). M-MDSC were purified either with magnetic beads (CD33⁺ HLA⁻DR^{-/low}) (see Figure 11B) or by cell sorting (CD33⁺CD14⁺HLA-DR^{-/low}) (see Figure 11C) and mixed with stimulated CFSE-labeled autologous T cells. Interestingly, M-MDSCs from both histological subtypes inhibited CD8⁺ and CD4⁺ T cell proliferation to the same extend. However, only M-MDSCs from SCC patients decreased substantially IL-2 and IFN-γ production from stimulated T cells in comparison to M-MDSCs from AC patients (see Figure 11A-B). These results show that M-MDSCs are not only increased in the blood of SCC patients but also exhibit higher suppressive activity on T cells than M-MDSC in blood from AC patients.

### References

AITKEN, A. S., ROY, D. G. & BOURGEOIS-DAIGNEAULT, M. C. 2017. Taking a Stab at Cancer; Oncolytic Virus-Mediated Anti-Cancer Vaccination Strategies. Biomedicines, 5.
BECHT, E., GIRALDO, N. A., LACROIX, L., BUTTARD, B., ELAROUCI, N., PETITPREZ, F., SELVES, J., LAURENT-PUIG, P., SAUTES-FRIDMAN, C., FRIDMAN, W. H. & DE REYNIES, A. 2016. Estimating the population abundance of tissue-infiltrating immune and stromal cell populations using gene expression. Genome Biol, 17, 218.
BIOCCA, S., ARCANGELI, T., TAGLIAFERRI, E., TESTA, B., VINDIGNI, G., OTERI, F., GIORGI, A., IACOVELLI, F., NOVELLI, G., DESIDERI, A. & FALCONI, M. 2013. Simulative and experimental investigation on the cleavage site that generates the soluble human LOX-1. Arch Biochem Biophys, 540, 9-18.
BLACK, C. C., TURK, M. J., DRAGNEV, K. & RIGAS, J. R. 2013. Adenocarcinoma contains more immune tolerance regulatory t-cell lymphocytes (versus squamous carcinoma) in non-small-cell lung cancer. Lung, 191, 265-70.
BRONTE, V., BRANDAU, S., CHEN, S. H., COLOMBO, M. P., FREY, A. B., GRETEN, T. F., MANDRUZZATO, S., MURRAY, P. J., OCHOA, A., OSTRAND-ROSENBERG, S., RODRIGUEZ, P. C., SICA, A., UMANSKY, V., VONDERHEIDE, R. H. & GABRILOVICH, D. I. 2016. Recommendations for myeloid-derived suppressor cell nomenclature and characterization standards. Nat Commun, 7, 12150.
CONDAMINE, T., DOMINGUEZ, G. A., YOUN, J. I., KOSSENKOV, A. V., MONY, S., ALICEA-TORRES, K., TCYGANOV, E., HASHIMOTO, A., NEFEDOVA, Y., LIN, C., PARTLOVA, S., GARFALL, A., VOGL, D. T., XU, X., KNIGHT, S. C., MALIETZIS, G., LEE, G. H., ERUSLANOV, E., ALBELDA, S. M., WANG, X., MEHTA, J. L., BEWTRA, M., RUSTGI, A., HOCKSTEIN, N., WITT, R., MASTERS, G., NAM, B., SMIRNOV, D., SEPULVEDA, M. A. & GABRILOVICH, D. I. 2016. Lectin-type oxidized LDL receptor-1 distinguishes population of human polymorphonuclear myeloid-derived suppressor cells in cancer patients. Sci Immunol, 1.
DERMAN, B. A., MILEHAM, K. F., BONOMI, P. D., BATUS, M. & FIDLER, M. J. 2015. Treatment of advanced squamous cell carcinoma of the lung: a review. Transl Lung Cancer Res, 4, 524-32.
DOMAGALA-KULAWIK, J., OSINSKA, I. & HOSER, G. 2014. Mechanisms of immune response regulation in lung cancer. Transl Lung Cancer Res, 3, 15-22.
DRAGHICIU, O., LUBBERS, J., NIJMAN, H. W. & DAEMEN, T. 2015. Myeloid derived suppressor cells-An overview of combat strategies to increase immunotherapy efficacy. Oncoimmunology, 4, e954829.
DUMITRU, C. A., MOSES, K., TRELLAKIS, S., LANG, S. & BRANDAU, S. 2012. Neutrophils and granulocytic myeloid-derived suppressor cells: immunophenotyping, cell biology and clinical relevance in human oncology. Cancer Immunol Immunother, 61, 1155-67.
GABRILOVICH, D. I. 2017. Myeloid-Derived Suppressor Cells. Cancer Immunol Res, 5, 3-8.
GABRILOVICH, D. I. & NAGARAJ, S. 2009. Myeloid-derived suppressor cells as regulators of the immune system. Nat Rev Immunol, 9, 162-74.
GABRILOVICH, D. I., OSTRAND-ROSENBERG, S. & BRONTE, V. 2012. Coordinated regulation of myeloid cells by tumours. Nat Rev Immunol, 12, 253-68.
HARROP, R. 2013. Cancer vaccines: Identification of biomarkers predictive of clinical efficacy. Human Vaccines & Immunotherapeutics, 9, 800-804.
HOUOT, R., SCHULTZ, L. M., MARABELLE, A. & KOHRT, H. 2015. T-cell-based Immunotherapy: Adoptive Cell Transfer and Checkpoint Inhibition. Cancer Immunol Res, 3, 1115-22.
HUANG, T., LI, J., ZHANG, C., HONG, Q., JIANG, D., YE, M. & DUAN, S. 2016. Distinguishing Lung Adenocarcinoma from Lung Squamous Cell Carcinoma by Two Hypomethylated and Three Hypermethylated Genes: A Meta-Analysis. PLoS ONE, 11, e0149088.
KO, H. J. & KIM, Y. J. 2016. Signal transducer and activator of transcription proteins: regulators of myeloid-derived suppressor cell-mediated immunosuppression in cancer. Arch Pharm Res, 39, 1597-1608.
KOEHN, B. H., APOSTOLOVA, P., HAVERKAMP, J. M., MILLER, J. S., MCCULLAR, V., TOLAR, J., MUNN, D. H., MURPHY, W. J., BRICKEY, W. J., SERODY, J. S., GABRILOVICH, D. I., BRONTE, V., MURRAY, P. J., TING, J. P., ZEISER, R. & BLAZAR, B. R. 2015. GVHD-associated, inflammasome-mediated loss of function in adoptively transferred myeloid-derived suppressor cells. Blood, 126, 1621-8.
LU, J., MITRA, S., WANG, X., KHAIDAKOV, M. & MEHTA, J. L. 2011a. Oxidative stress and lectin-like ox-LDL-receptor LOX-1 in atherogenesis and tumorigenesis. Antioxid Redox Signal, 15, 2301-33.
LU, T., RAMAKRISHNAN, R., ALTIOK, S., YOUN, J. I., CHENG, P., CELIS, E., PISAREV, V., SHERMAN, S., SPORN, M. B. & GABRILOVICH, D. 2011b. Tumor-infiltrating myeloid cells induce tumor cell resistance to cytotoxic T cells in mice. J Clin Invest, 121, 4015-29.
MALEK, E., DE LIMA, M., LETTERIO, J. J., KIM, B. G., FINKE, J. H., DRISCOLL, J. J. & GIRALT, S. A. 2016. Myeloid-derived suppressor cells: The green light for myeloma immune escape. Blood Rev, 30, 341-8.
MARIGO, I., BOSIO, E., SOLITO, S., MESA, C., FERNANDEZ, A., DOLCETTI, L., UGEL, S., SONDA, N., BICCIATO, S., FALISI, E., CALABRESE, F., BASSO, G., ZANOVELLO, P., COZZI, E., MANDRUZZATO, S. & BRONTE, V. 2010. Tumor-induced tolerance and immune suppression depend on the C/EBPbeta transcription factor. Immunity, 32, 790-802.
MCNAMARA, M. A., NAIR, S. K. & HOLL, E. K. 2015. RNA-Based Vaccines in Cancer Immunotherapy. J Immunol Res, 2015, 794528.
MOLON, B., UGEL, S., DEL POZZO, F., SOLDANI, C., ZILIO, S., AVELLA, D., DE PALMA, A., MAURI, P., MONEGAL, A., RESCIGNO, M., SAVINO, B., COLOMBO, P., JONJIC, N., PECANIC, S., LAZZARATO, L., FRUTTERO, R., GASCO, A., BRONTE, V. & VIOLA, A. 2011. Chemokine nitration prevents intratumoral infiltration of antigen-specific T cells. J Exp Med, 208, 1949-62.
NAGARAJ, S., GUPTA, K., PISAREV, V., KINARSKY, L., SHERMAN, S., KANG, L., HERBER, D. L., SCHNECK, J. & GABRILOVICH, D. I. 2007. Altered recognition of antigen is a mechanism of CD8+ T cell tolerance in cancer. Nat Med, 13, 828-35.
RAMAKRISHNAN, R., ASSUDANI, D., NAGARAJ, S., HUNTER, T., CHO, H. I., ANTONIA, S., ALTIOK, S., CELIS, E. & GABRILOVICH, D. I. 2010. Chemotherapy enhances tumor cell susceptibility to CTL-mediated killing during cancer immunotherapy in mice. J Clin Invest, 120, 1111-24.
SCAPINI, P. & CASSATELLA, M. A. 2014. Social networking of human neutrophils within the immune system. Blood, 124, 710-9.
TALMADGE, J. E. & GABRILOVICH, D. I. 2013. History of myeloid-derived suppressor cells. Nat Rev Cancer, 13, 739-52.
TRAVIS, W. D., BRAMBILLA, E., NICHOLSON, A. G., YATABE, Y., AUSTIN, J. H., BEASLEY, M. B., CHIRIEAC, L. R., DACIC, S., DUHIG, E., FLIEDER, D. B., GEISINGER, K., HIRSCH, F. R., ISHIKAWA, Y., KERR, K. M., NOGUCHI, M., PELOSI, G., POWELL, C. A., TSAO, M. S., WISTUBA, I. & PANEL, W. H. O. 2015. The 2015 World Health Organization Classification of Lung Tumors: Impact of Genetic, Clinical and Radiologic Advances Since the 2004 Classification. J Thorac Oncol, 10, 1243-60.
TRAVIS, W. D., BRAMBILLA, E., NOGUCHI, M., NICHOLSON, A. G., GEISINGER, K., YATABE, Y., ISHIKAWA, Y., WISTUBA, I., FLIEDER, D. B., FRANKLIN, W., GAZDAR, A., HASLETON, P. S., HENDERSON, D. W., KERR, K. M., PETERSEN, I., ROGGLI, V., THUNNISSEN, E. & TSAO, M. 2013. Diagnosis of Lung Cancer in Small Biopsies and Cytology: Implications of the 2011 International Association for the Study of Lung Cancer/American Thoracic Society/European Respiratory Society Classification. Archives of pathology & laboratory medicine, 137, 668-684.
TURNIS, M. E. & ROONEY, C. M. 2010. Enhancement of dendritic cells as vaccines for cancer. Immunotherapy, 2, 847-62.
UMANSKY, V., BLATTNER, C., GEBHARDT, C. & UTIKAL, J. 2016. The Role of Myeloid-Derived Suppressor Cells (MDSC) in Cancer Progression. Vaccines (Basel), 4.
WALTER, S., WEINSCHENK, T., STENZL, A., ZDROJOWY, R., PLUZANSKA, A., SZCZYLIK, C., STAEHLER, M., BRUGGER, W., DIETRICH, P. Y., MENDRZYK, R., HILF, N., SCHOOR, O., FRITSCHE, J., MAHR, A., MAURER, D., VASS, V., TRAUTWEIN, C., LEWANDROWSKI, P., FLOHR, C., POHLA, H., STANCZAK, J. J., BRONTE, V., MANDRUZZATO, S., BIEDERMANN, T., PAWELEC, G., DERHO VANES SIAN, E., YAMAGISHI, H., MIKI, T., HONGO, F., TAKAHA, N., HIRAKAWA, K., TANAKA, H., STEVANOVIC, S., FRISCH, J., MAYER-MOKLER, A., KIRNER, A., RAMMENSEE, H. G., REINHARDT, C. & SINGH-JASUJA, H. 2012. Multipeptide immune response to cancer vaccine IMA901 after single-dose cyclophosphamide associates with longer patient survival. Nat Med, 18, 1254-61.
WESOLOWSKI, R., MARKOWITZ, J. & CARSON, W. E., 3RD 2013. Myeloid derived suppressor cells - a new therapeutic target in the treatment of cancer. J Immunother Cancer, 1, 10.
XIAO, Y. F., JIE, M. M., LI, B. S., HU, C. J., XIE, R., TANG, B. & YANG, S. M. 2015. Peptide-Based Treatment: A Promising Cancer Therapy. J Immunol Res, 2015, 761820.
YANG, B., JEANG, J., YANG, A., WU, T. C. & HUNG, C. F. 2014. DNA vaccine for cancer immunotherapy. Hum Vaccin Immunother, 10, 3153-64.
WO 2013/004708
WO 2015/097037

## Claims

1. A composition comprising a dendritic cell therapy/vaccine (DC vaccine) for use in treating or delaying progression of squamous cell carcinoma (SCC) of the lungs in an individual, wherein the treatment comprises
administration of the composition in combination with a second composition comprising
a myeloid-derived suppressor cell (MDSC)-inhibiting agent and an optional pharmaceutically acceptable carrier,
wherein the DC vaccine has been prepared with an antigen source selected from tumor associated peptide(s), whole antigens from DNA or RNA, whole antigen-protein, idiotype protein, tumor lysate/whole tumor cells or viral vector-delivered whole antigen,
wherein the MDSC-inhibiting agent blocks or inhibits differentiation/maturation of MDSCs, blocks or inhibits migration of MDSCs or induces depletion of MDSCs/apoptosis of MDSCs, or inhibits expansion of MDSCs, or inhibits MDSC effector functions, and
wherein the MDSC-inhibiting agent that blocks or inhibits differentiation/maturation of MDSCs is selected from all-trans-retinoic acid, Curcumin derivatives; wherein the inhibitor of migration of MDSCs is selected from Zolendronic acid, anti-glycan antibodies and CSF-1R inhibitors; wherein the inducer of depletion or apoptosis of MDSCs is selected from tyrosine kinase inhibitors, preferably Sunitinib, anti-Gr1 antibodies, IL4Rα aptamer, Gemcitabine, Cisplatin, Paclitaxel, 17-Dimethylaminoethylamino-17-demethoxygeldanamycin (17-DMAG) or 5-fluorouracil (5-FU); wherein the inhibitor of expansion of MDSCs is selected from Bevacizumab, Celecoxib and Pimozide; and wherein the inhibitor of MDSC effector functions is an inducer of oxidative stress and is selected from N-hydroxyl-L-Arginine (NOHA), Nitroaspirin, N-acetyl cysteine (NAC), CpG oligodeoxy-nucleotides, Bardoxolone methyl (CDDO-Me), Withaferin A or Stattic.

2. The composition according to claim 1, wherein the individual is **characterized in that**
- the fraction of living cells in peripheral blood mononuclear cells which are monocytic MDSCs (M-MDSCs) is at least 0.08%, preferably at least 0.1%,
- the fraction of living cells in tumor tissue which are M-MDSCs, is at least 0.005%, preferably at least 0.008%,
- the level of LOX-1 in serum or plasma is above 75 pg/ml, preferentially above 100 pg/ml.
- the percentage of LOX-1-expressing polymorphonuclear MDSCs (PMN-MDSC) among all PMN-MDSC is at least 5%, preferentially 10%.
- the fraction of living cells in tumor tissue which are PMN-MDSCs is at least 2%, preferably at least 2.5%,
- the fraction of living Treg cells in peripheral blood mononuclear cells is at most 1.8%, preferably at most 1.5%,
- the fraction of living Treg cells in tumor tissue is at most 10% of CD4⁺ T cells, preferably at most 9% of CD4⁺ T cells,
- the fraction of CD3ζ among CD4⁺ T cells in peripheral blood mononuclear cells is reduced compared to control CD4⁺ T cells of healthy individuals by a factor of at least 0.9, preferably at least by a factor of 0.8,
- the fraction of CD3ζ among CD8⁺ T cells in peripheral blood mononuclear cells is reduced compared to control CD8⁺ T cells of healthy individuals by a factor of at least 0.75, preferably at least by a factor of 0.65, and/or
- the relative expression of Arginase 1 in peripheral blood mononuclear cells of patients compared to control peripheral blood mononuclear cells of healthy individuals is increased at least by a factor of 2.5.

3. A composition comprising a dendritic cell therapy/vaccine (DC vaccine) for use in treating or delaying progression of non-small cell lung cancer (NSCLC) in an individual, wherein the treatment comprises
administration of the composition in combination with a second composition comprising
a myeloid-derived suppressor cell (MDSC)-inhibiting agent and an optional pharmaceutically acceptable carrier
wherein the individual is **characterized by** a immunosuppressive tumor microenvironment caused by the presence of MDSCs,
wherein the DC vaccine has been prepared with an antigen source selected from tumor associated peptide(s), whole antigens from DNA or RNA, whole antigen-protein, idiotype protein, tumor lysate/whole tumor cells or viral vector-delivered whole antigen,
wherein the individual with a immunosuppressive tumor microenvironment is further **characterized in that**
- the fraction of living cells in peripheral blood mononuclear cells which are monocytic MDSCs (M-MDSCs) is at least 0.08%, preferably at least 0.1%,
- the fraction of living cells in tumor tissue which are M-MDSCs, is at least 0.005%, preferably at least 0.008%,
- the level of LOX-1 in serum or plasma is above 75 pg/ml, preferentially above 100 pg/ml.
- the percentage of LOX-1-expressing PMN-MDSC among all PMN-MDSC is at least 5%, preferentially 10%.
- the fraction of living cells in tumor tissue which are PMN-MDSCs is at least 2%, preferably at least 2.5%,
- the fraction of living Treg cells in peripheral blood mononuclear cells is at most 1.8%, preferably at most 1.5%,
- the fraction of living Treg cells in tumor tissue is at most 10% of CD4⁺ cells, preferably at most 9% of CD4⁺ cells,
- the fraction of CD3ζ among CD4⁺ T cells in peripheral blood mononuclear cells is reduced compared to control cells of healthy individuals by a factor of at least 0.9, preferably at least by a factor of 0.8,)
- the fraction of CD3ζ among CD8⁺ T cells in peripheral blood mononuclear cells is reduced compared to control cells of healthy individuals by a factor of at least 0.75, preferably at least by a factor of 0.65, and/or
- the relative expression of Arginase 1 in peripheral blood mononuclear cells of patients compared to control peripheral blood mononuclear cells of healthy individuals is increased at least by a factor of 2.5,
wherein the MDSC-inhibiting agent blocks or inhibits differentiation/maturation of MDSCs, blocks or inhibits migration of MDSCs or induces depletion of MDSCs/apoptosis of MDSCs, or inhibits expansion of MDSCs, or inhibits MDSC effector functions, and
wherein the MDSC-inhibiting agent that blocks or inhibits differentiation/maturation of MDSCs is selected from all-trans-retinoic acid, Curcumin derivatives; wherein the inhibitor of migration of MDSCs is selected from Zolendronic acid, anti-glycan antibodies and CSF-1R inhibitors; wherein the inducer of depletion or apoptosis of MDSCs is selected from tyrosine kinase inhibitors, preferably Sunitinib, anti-Gr1 antibodies, IL4Rα aptamer, Gemcitabine, Cisplatin, Paclitaxel, 17-Dimethylaminoethylamino-17-demethoxygeldanamycin (17-DMAG) or 5-fluorouracil (5-FU); wherein the inhibitor of expansion of MDSCs is selected from Bevacizumab, Celecoxib and Pimozide; and wherein the inhibitor of MDSC effector functions is an inducer of oxidative stress and is selected from N-hydroxyl-L-Arginine (NOHA), Nitroaspirin, N-acetyl cysteine (NAC), CpG oligodeoxy-nucleotides, Bardoxolone methyl (CDDO-Me), Withaferin A or Stattic.

4. The composition of claim 2 or 3, wherein M-MDSCs have a CD14⁺CD15⁻CD33^{hi}HLA-DR^{-/lo} phenotype.

5. The composition according to claims 2-4, wherein the PMN-MDSCs have a suppressive CD14-CD15⁺CD11b⁺ phenotype, preferably expressing LOX-1.

6. The composition according to claims 1, 2, 4 or 5, wherein the individual has been diagnosed with SCC preferably SCC of the lungs.

7. The composition according to claim 1, wherein the whole tumor cells have been prepared by high hydrostatic pressure.

8. The composition according to claim 1 or claim 3, wherein second composition comprises carboplatin plus paclitaxel for the treatment of stage IV NSCLC, pemetrexed plus carboplatin for the treatment of stage IV EGFR wild-type NSCLC, gemcitabine plus cisplatin or pemetrexed plus cisplatin for the treatment of stage IIIB to IV EGFR wild-type NSCLC or vinorelbine plus carboplatin for the treatment of stage IIA-IV NSCLC.

## Patentansprüche

1. Zusammensetzung umfassend eine dendritische Zell-Therapie/-Vakzin (DC-Vakzin) zur Verwendung bei der Behandlung oder Verzögerung des Fortschreitens eines Plattenepithelkarzinoms (SCC) der Lunge in einem Individuum, wobei die Behandlung die Verabreichung der Zusammensetzung in Kombination mit einer zweiten Zusammensetzung umfasst, die
ein von myeloiden Suppressorzellen (MDSC) abgeleitetes inhibierendes Mittel und einen optionalen pharmazeutisch akzeptablen Träger umfasst,
wobei das DC-Vakzin mit einer Antigenquelle hergestellt wurde, ausgewählt aus tumorassoziierten Peptid(en), ganzen Antigenen aus DNA oder RNA, ganzen Antigen-Proteinen, idiotypischen Proteinen, Tumorlysat/ganzen Tumorzellen oder durch virale Vektoren bereitgestellten ganzen Antigenen,
wobei das MDSC-inhibierende Mittel die Differenzierung/Reifung von MDSCs blockiert oder hemmt, die Migration von MDSCs blockiert oder hemmt oder die Depletion von MDSCs/Apoptose von MDSCs induziert oder die Expansion von MDSCs hemmt oder die Effektorfunktionen von MDSCs hemmt, und
wobei das MDSC-inhibierende Mittel, das die Differenzierung/Reifung von MDSCs blockiert oder hemmt, ausgewählt ist aus all-trans-Retinsäure, Curcumin-Derivaten; wobei der Inhibitor der Migration von MDSCs ausgewählt ist aus Zolendronsäure, Anti-Glykan-Antikörpern und CSF-1R-Inhibitoren; wobei der Auslöser der Depletion oder Apoptose von MDSCs ausgewählt ist aus Tyrosinkinaseinhibitoren, vorzugsweise Sunitinib, Anti-Gr1-Antikörpern, IL4Rα Aptamer, Gemcitabin, Cisplatin, Paclitaxel, 17-Dimethylaminoethylamino-17-demethoxygeldanamycin (17-DMAG) oder 5-Fluorouracil (5-FU); wobei der Inhibitor der Expansion von MDSCs ausgewählt ist aus Bevacizumab, Celecoxib und Pimozid; und wobei der Inhibitor der MDSC-Effektorfunktionen ein Auslöser von oxidativem Stress ist und ausgewählt ist aus N-Hydroxyl-L-Arginin (NOHA), Nitroaspirin, N-Acetylcystein (NAC), CpG-Oligodesoxy-Nukleotiden, Bardoxolon-Methyl (CDDO-Me), Withaferin A oder Stattic.

2. Zusammensetzung nach Anspruch 1, wobei das Individuum **dadurch gekennzeichnet ist, dass**
- der Anteil der lebenden Zellen in den mononukleären Zellen des peripheren Blutes, bei denen es sich um monozytäre MDSCs (M-MDSCs) handelt, mindestens 0,08 %, vorzugsweise mindestens 0,1 % beträgt,
- der Anteil der lebenden Zellen im Tumorgewebe, bei denen es sich um M-MDSCs handelt, mindestens 0,005 %, vorzugsweise mindestens 0,008 % beträgt,
- das LOX-1-Level im Serum oder Plasma über 75 pg/ml, vorzugsweise über 100 pg/ml liegt.
- der Prozentsatz der LOX-1-exprimierenden polymorphkernigen MDSC (PMN-MDSC) unter allen PMN-MDSC mindestens 5 %, vorzugsweise 10 % beträgt.
- der Anteil der lebenden Zellen im Tumorgewebe, bei denen es sich um PMN-MDSCs handelt, mindestens 2 %, vorzugsweise mindestens 2,5 % beträgt,
- der Anteil der lebenden Treg-Zellen in den mononukleären Zellen des peripheren Blutes höchstens 1,8 %, vorzugsweise höchstens 1,5 % beträgt,
- der Anteil der lebenden Treg-Zellen im Tumorgewebe höchstens 10 % der CD4⁺ T-Zellen, vorzugsweise höchstens 9 % der CD4⁺ T-Zellen beträgt,
- der Anteil von CD3ζ unter den CD4⁺ T-Zellen in den mononukleären Zellen des peripheren Blutes im Vergleich zu den Kontroll-CD4⁺ T-Zellen gesunder Individuuen um einen Faktor von mindestens 0,9, vorzugsweise mindestens um einen Faktor von 0,8, reduziert ist,
- der Anteil von CD3ζ unter den CD8⁺ T-Zellen in den mononukleären Zellen des peripheren Blutes im Vergleich zu den Kontroll-CD8⁺ T-Zellen gesunder Individuen um einen Faktor von mindestens 0,75, vorzugsweise mindestens um einen Faktor von 0,65, reduziert ist und/oder
- die relative Expression von Arginase 1 in mononukleären Zellen des peripheren Blutes von Patienten im Vergleich zu mononukleären Zellen des peripheren Blutes von gesunden Individuen mindestens um den Faktor 2,5 erhöht ist.

3. Zusammensetzung umfassend eine dendritische Zell-Therapie/-Vakzin (DC-Vakzin) zur Verwendung bei der Behandlung oder Verzögerung des Fortschreitens von nicht-kleinzelligem Lungenkrebs (NSCLC) in einem Individuum, wobei die Behandlung die Verabreichung der Zusammensetzung in Kombination mit einer zweiten Zusammensetzung umfasst, die
ein von myeloiden Suppressorzellen (MDSC) abgeleitetes inhibierendes Mittel und einen optionalen pharmazeutisch akzeptablen Träger umfasst, wobei das Individuum durch eine immunsuppressive Tumormikroumgebung gekennzeichnet ist, die durch die Anwesenheit von MDSCs verursacht wird,
wobei das DC-Vakzin mit einer Antigenquelle hergestellt wurde, ausgewählt aus tumorassoziierten Peptid(en), ganzen Antigenen aus DNA oder RNA, ganzen Antigen-Proteinen, idiotypischen Proteinen, Tumorlysat/ganzen Tumorzellen oder durch virale Vektoren bereitgestellten ganzen Antigenen,
wobei das Individuum mit einer immunsuppressiven Tumormikroumgebung weiterhin **dadurch gekennzeichnet ist, dass**
- der Anteil der lebenden Zellen in den mononukleären Zellen des peripheren Blutes, bei denen es sich um monozytäre MDSCs (M-MDSCs) handelt, mindestens 0,08 %, vorzugsweise mindestens 0,1 % beträgt,
- der Anteil der lebenden Zellen im Tumorgewebe, bei denen es sich um M-MDSCs handelt, mindestens 0,005 %, vorzugsweise mindestens 0,008 % beträgt,
- das LOX-1-Level im Serum oder Plasma über 75 pg/ml, vorzugsweise über 100 pg/ml liegt.
- der Prozentsatz der LOX-1-exprimierenden PMN-MDSC unter allen PMN-MDSC mindestens 5 %, vorzugsweise 10 % beträgt.
- der Anteil der lebenden Zellen im Tumorgewebe, bei denen es sich um PMN-MDSCs handelt, mindestens 2 %, vorzugsweise mindestens 2,5 % beträgt,
- der Anteil der lebenden Treg-Zellen in den mononukleären Zellen des peripheren Blutes höchstens 1,8 %, vorzugsweise höchstens 1,5 % beträgt,
- der Anteil der lebenden Treg-Zellen im Tumorgewebe höchstens 10 % der CD4⁺ Zellen, vorzugsweise höchstens 9 % der CD4⁺ Zellen beträgt,
- der Anteil der CD3ζ unter den CD4⁺ T-Zellen in den mononukleären Zellen des peripheren Blutes im Vergleich zu den Kontrollzellen gesunder Personen um einen Faktor von mindestens 0,9, vorzugsweise mindestens um einen Faktor von 0,8, reduziert ist)
- der Anteil der CD3ζ unter den CD8⁺ T-Zellen in den mononukleären Zellen des peripheren Blutes im Vergleich zu den Kontrollzellen gesunder Individuen um einen Faktor von mindestens 0,75, vorzugsweise mindestens um einen Faktor von 0,65, reduziert ist und/oder
- die relative Expression von Arginase 1 in mononukleären Zellen des peripheren Blutes von Patienten im Vergleich zu mononukleären Zellen des peripheren Blutes von gesunden Individuen mindestens um den Faktor 2,5 erhöht ist,
wobei das MDSC-inhibierende Mittel die Differenzierung/Reifung von MDSCs blockiert oder hemmt, die Migration von MDSCs blockiert oder hemmt oder die Depletion von MDSCs/Apoptose von MDSCs induziert oder die Expansion von MDSCs hemmt oder die Effektorfunktionen von MDSCs hemmt, und
wobei das MDSC-inhibierende Mittel, das die Differenzierung/Reifung von MDSCs blockiert oder hemmt, ausgewählt ist aus all-trans-Retinsäure, Curcumin-Derivaten; wobei der Inhibitor der Migration von MDSCs ausgewählt ist aus Zolendronsäure, Anti-Glykan-Antikörpern und CSF-1R-Inhibitoren; wobei der Auslöser der Depletion oder Apoptose von MDSCs ausgewählt ist aus Tyrosinkinaseinhibitoren, vorzugsweise Sunitinib, Anti-Gr1-Antikörpern, IL4Rα Aptamer, Gemcitabin, Cisplatin, Paclitaxel, 17-Dimethylaminoethylamino-17-demethoxygeldanamycin (17-DMAG) oder 5-Fluorouracil (5-FU); wobei der Inhibitor der Expansion von MDSCs ausgewählt ist aus Bevacizumab, Celecoxib und Pimozid; und wobei der Inhibitor der MDSC-Effektorfunktionen ein Induktor von oxidativem Stress ist und ausgewählt ist aus N-Hydroxyl-L-Arginin (NOHA), Nitroaspirin, N-Acetylcystein (NAC), CpG-Oligodesoxy-Nukleotiden, Bardoxolon-Methyl (CDDO-Me), Withaferin A oder Stattic.

4. Die Zusammensetzung nach Anspruch 2 oder 3, wobei M-MDSCs einen CD14⁺ CD15⁻ CD33^{hi}HLA-DR^{-/lo} Phänotyp aufweisen.

5. Zusammensetzung nach den Ansprüchen 2 bis 4, wobei die PMN-MDSCs einen suppressiven CD14⁻ CD15⁺ CD11b⁺ Phänotyp aufweisen und vorzugsweise LOX-1 exprimieren.

6. Die Zusammensetzung nach Anspruch 1, 2, 4 oder 5, wobei bei dem Individuum SCC, vorzugsweise SCC der Lunge, diagnostiziert wurde.

7. Zusammensetzung nach Anspruch 1, wobei die ganzen Tumorzellen durch hohen hydrostatischen Druck hergestellt wurden.

8. Zusammensetzung nach Anspruch 1 oder Anspruch 3, wobei die zweite Zusammensetzung Carboplatin plus Paclitaxel für die Behandlung von NSCLC im Stadium IV, Pemetrexed plus Carboplatin für die Behandlung von NSCLC vom EGFR-Wildtyp im Stadium IV, Gemcitabin plus Cisplatin oder Pemetrexed plus Cisplatin für die Behandlung von NSCLC vom EGFR-Wildtyp im Stadium IIIB bis IV oder Vinorelbin plus Carboplatin für die Behandlung von NSCLC im Stadium IIA-IV umfasst.

## Revendications

1. Composition comprenant un vaccin/thérapie cellulaire dendritique (vaccin dendritic cell, DC) destinée à être utilisé dans le traitement ou le retard de la progression du carcinome cellulaire squameux (squamous cell carcinoma, SCC) des poumons chez un individu, dans laquelle le traitement comprend l'administration de la composition en combinaison avec une seconde composition comprenant un agent inhibiteur de cellules myéloïdes suppressives (myeloid-derived suppressor cell, MDSC) et d'un véhicule pharmaceutiquement acceptable optionnel,
dans laquelle le vaccin DC a été préparé avec une source d'antigène choisie parmi un(des) peptide(s) associé(s) à une tumeur, des antigènes entiers d'ADN ou d'ARN, une protéine d'antigène entier, des cellules tumorales entières/lysat cellulaire ou un antigène entier délivré par un vecteur viral,
dans laquelle l'agent inhibiteur de MDSC bloque ou inhibe la différenciation/maturation de MDSC, bloque ou inhibe la migration de MDSC ou induit la diminution de MDSC/l'apoptose de MDSC ou inhibe l'expansion de MDSC ou inhibe les fonctions effectrices de MDSC et
dans laquelle l'agent inhibiteur de MDSC qui bloque ou inhibe la différenciation/maturation de MDSC est choisi parmi l'acide entièrement-trans-rétinoïque, les dérivés du curcumin ; dans laquelle l'inhibiteur de migration de MDSC est choisi parmi l'acide zolendronique, les anticorps anti-glycane et les inhibiteurs de CSF-1R ; dans laquelle l'inducteur de diminution ou d'apoptose de MDSC est choisi parmi les inhibiteurs de tyrosine kinase, de préférence le sunitinib, les anticorps anti-Gr1, l'aptamère IL4Rα, la gemcitabine, le cisplatine, le paclitaxel, la 17-diméthylaminoéthylamino-17-déméthoxygeldanamycine (17-DMAG) ou le 5-fluorouracile (5-FU) ; dans laquelle l'inhibiteur de l'expansion de MDSC est choisi parmi le bévacizumab, le célécoxib et le pimozide ; et dans laquelle l'inhibiteur des fonctions effectrices de MDSC est un inducteur de stress oxydatif et est choisi parmi la N-hydroxyl-L-arginine (NOHA), la nitroaspirine, la N-acétyl cystéine (NAC), les oligodésoxynucléotides CpG, la bardoxolone méthyle (CDDO-Me), la withaférine A ou le stattic.

2. Composition selon la revendication 1, dans laquelle l'individu est **caractérisé en ce que**
- la fraction de cellules vivantes dans les cellules mononucléaire du sang périphérique qui sont des MDSC monocytaires (M-MDSC) est d'au moins 0,08 %, de préférence d'au moins 0,1 %,
- la fraction de cellules vivantes dans le tissu tumoral qui sont des M-MDSC, est d'au moins 0,005 %, de préférence d'au moins 0,008 %,
- le niveau de LOX-1 dans le sérum ou le plasma est au-dessus de 75 pg/ml, préférentiellement au-dessus de 100 pg/ml,
- le pourcentage de MDSC polymorphonucléaires (PMN-MDSC) exprimant LOX-1 parmi tous les PMN-MDSC est d'au moins 5 %, préférentiellement de 10 %,
- la fraction de cellules vivantes dans le tissu tumoral qui sont des PMN-MDSC est d'au moins 2 %, de préférence d'au moins 2,5 %,
- la fraction de lymphocytes Treg vivants dans les cellules mononucléaires du sang périphérique est au plus de 1,8 %, de préférence au plus de 1,5 %,
- la fraction de lymphocytes Treg vivants dans le tissu tumoral est au plus de 10 % de lymphocytes T CD4⁺, de préférence au plus de 9 % de lymphocytes T CD4⁺,
- la fraction de CD3ζ parmi les lymphocytes T CD4⁺ dans les cellules mononucléaires du sang périphérique est réduite par rapport aux lymphocytes CD4⁺ témoins d'individus sains par un facteur d'au moins 0,9, de préférence d'au moins par un facteur de 0,8,
- la fraction de CD3ζ parmi les lymphocytes T CD8⁺ dans les cellules mononucléaires du sang périphérique est réduite par rapport aux lymphocytes CD8⁺ témoins d'individus sains par un facteur d'au moins 0,75, de préférence d'au moins par un facteur de 0,65 et/ou
- l'expression relative de l'arginase 1 dans les cellules mononucléaires du sang périphérique de patients par rapport aux cellules mononucléaires du sang périphérique témoins d'individus sains est augmentée au moins par un facteur de 2,5.

3. Composition comprenant un vaccin/thérapie cellulaire dendritique (vaccin dendritic cell, DC) destinée à être utilisée dans le traitement ou le retard de la progression du cancer du poumon à cellules non petites (non-small cell lung cancer, NSCLC) chez un individu, dans laquelle le traitement comprend l'administration de la composition en combinaison avec une seconde composition comprenant un agent inhibiteur de cellules myéloïdes suppressives (myeloid-derived suppressor cell, MDSC) et un véhicule pharmaceutiquement acceptable optionnel
dans laquelle l'individu est **caractérisé par** un microenvironnement de tumeur immunosuppressif provoqué par la présence de MDSC,
dans laquelle le vaccin DC a été préparé avec une source d'antigène choisie parmi un (des) peptide(s) associé(s) à une tumeur, des antigènes entiers d'ADN ou d'ARN, une protéine d'antigène entier, une protéine idiotype, un lysat tumoral/cellules tumorales entières ou un antigène entier délivré par un vecteur viral,
dans laquelle l'individu avec un microenvironnement tumoral immunosuppressif est en outre **caractérisé en ce que**
- la fraction de cellules vivantes dans les cellules mononucléaires du sang périphérique qui sont des MDSC monocytaires (M-MDSC) est d'au moins 0,08 %, de préférence d'au moins 0,1 %,
- la fraction de cellules vivantes dans le tissu tumoral qui sont des MDSC est d'au moins 0,005 %, de préférence d'au moins 0,008 %,
- le niveau de LOX-1 dans le sérum ou le plasma est au-dessus de 75 pg/ml, préférentiellement au-dessus de 100 pg/ml,
- le pourcentage de PMN-MDSC exprimant LOX-1 parmi toutes les PMN-MDSC est d'au moins 5%, préférentiellement de 10 %,
- la fraction de cellules vivantes dans le tissu tumoral qui sont des PMN-MDSC est d'au moins 2 %, de préférence d'au moins 2,5 %,
- la fraction de lymphocytes Treg vivants dans les cellules mononucléaires du sang périphérique est au plus de 1,8 %, de préférence au plus de 1,5 %,
- la fraction de lymphocytes Treg vivants dans le tissu tumoral est d'au plus 10 % de lymphocytes CD4⁺, de préférence au plus de 9 % de lymphocytes CD4⁺,
- la fraction de **CD3ζ** parmi les lymphocytes T CD4⁺ dans les cellules mononucléaires du sang périphérique est réduite par rapport aux cellules témoins d'individus sains par un facteur d'au moins 0,9, de préférence d'au moins par un facteur de 0,8,
- la fraction de **CD3ζ** parmi les lymphocytes T CD8⁺ dans les cellules mononucléaires du sang périphérique est réduite par rapport aux cellules témoins d'individus sains par un facteur d'au moins 0,75, de préférence d'au moins par un facteur de 0,65 et/ou
- l'expression relative de l'arginase 1 dans les cellules mononucléaires du sang périphérique de patients par rapport aux cellules mononucléaires du sang périphérique témoins d'individus sains est augmentée d'au moins par un facteur de 2,5,
dans laquelle l'agent inhibiteur de MDSC bloque ou inhibe la différenciation/maturation de MDSC ou bloque ou inhibe la migration de MDSC ou induit la diminution de MDSC/l'apoptose de MDSC ou inhibe l'expansion de MDSC ou inhibe les fonctions effectrices de MDSC et
dans laquelle l'agent inhibiteur de MDSC qui bloque ou inhibe la différenciation/maturation de MDSC est choisi parmi l'acide entièrement-trans-rétinoïque, les dérivés du curcumin ; dans laquelle l'inhibiteur de migration de MDSC est choisi parmi l'acide zolendronique, les anticorps anti-glycane et les inhibiteurs de CSF-1R ; dans laquelle l'inducteur de diminution ou d'apoptose de MDSC est choisi parmi les inhibiteurs de tyrosine kinase, de préférence le sunitinib, les anticorps anti-Gr1, l'aptamère IL4Rα, la gemcitabine, le cisplatine, le paclitaxel, la 17-diméthylaminoéthylamino-17-déméthoxygeldanamycine (17-DMAG) ou le 5-fluorouracile (5-FU) ; dans laquelle l'inhibiteur de l'expansion de MDSC est choisi parmi le bévacizumab, le célécoxib et le pimozide ; et dans laquelle l'inhibiteur des fonctions effectrices de MDSC est un inducteur de stress oxydatif et est choisi parmi la N-hydroxyl-L-arginine (NOHA), la nitroaspirine, la N-acétyl cystéine (NAC), les oligodésoxynucléotides CpG, la bardoxolone méthyle (CDDO-Me), la withaférine A ou le stattic.

4. Composition selon la revendication 2 ou 3, dans laquelle les M-MDSC ont un phénotype CD14⁺CD15-CD33^{hi}HLA-DR^{-/lo}.

5. Composition selon les revendications 2 à 4, dans laquelle les PMN-MDSC ont un phénotype suppresseur CD14⁻CD15⁺CD11b⁺, de préférence exprimant LOX-1.

6. Composition selon les revendications 1, 2, 4 ou 5, dans laquelle l'individu a été diagnostiqué avec un SCC de préférence un SCC des poumons.

7. Composition selon la revendication 1, dans laquelle les cellules tumorales entières ont été préparées par pression hydrostatique élevée.

8. Composition selon la revendication 1 ou la revendication 3, dans laquelle la seconde composition comprend du carboplatine plus du pacliitaxel pour le traitement du NSCLC de stade IV, du pémétrexed plus du carboplatine pour le traitement du NSCLC sauvage d'EGFR de stade IV, de la gemcitabine plus du cisplatine ou du pémétrexed plus du cisplatine pour le traitement du NSCLC sauvage d'EGFR de stade IIIb à IV ou de la vinorelbine plus du carboplatine pour le traitement du NSCLC de stade IIA à IV.
